(19)

**Europäisches Patentamt**
**European Patent Office**
**Office européen des brevets**

(11)  **EP 4 438 050 A1**

(12)  # EUROPEAN PATENT APPLICATION

(43) Date of publication:
**02.10.2024  Bulletin 2024/40**

(21) Application number: **23382314.5**

(22) Date of filing: **31.03.2023**

(51) International Patent Classification (IPC):
*A61K 35/74* (2015.01)     *A23L 33/135* (2016.01)
*A61K 9/00* (2006.01)      *A61K 35/742* (2015.01)
*A61K 35/745* (2015.01)    *A61K 35/747* (2015.01)
*A61P 35/00* (2006.01)

(52) Cooperative Patent Classification (CPC):
**A61K 35/74; A23L 33/135; A61K 9/0095;**
**A61K 9/146; A61K 35/742; A61K 35/745;**
**A61K 35/747; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Igen Biolab Group AG**
**6300 Zug (CH)**

(72) Inventors:
• **MOSCOSO DEL PRADO UCELAY, Juan**
**28760 Tres Cantos (ES)**

• **PEREZAGUA MARIN, Carmen**
**28055 Madrid (ES)**
• **USAN GRACIA, Luis ALberto**
**28045 Madrid (ES)**
• **GUARDIA ALBA, María Jesús**
**18198 Granada (ES)**

(74) Representative: **Padial Martinez, Ana Belen**
**Baylos 5.0 Legal Advisors S.L.**
**Av. Diagonal 435, 1.1**
**08008 Barcelona (ES)**

(54)  **POSTBIOTIC COMPOSITION COMPRISING BACTERIAL LYSATES FOR ORAL ADMINISTRATION FOR TREATING SOLID TUMORS**

(57)    The present invention discloses a postbiotic composition for oral administration for use in the treatment of solid tumors in a patient, said composition comprising lysates of probiotic microorganisms of the genera *Bacillus*; *Bifidobacterium*; *Lactobacillus, Saccharomyces, Streptococcus.*

EP 4 438 050 A1

Description

## TECHNICAL FIELD OF THE INVENTION

The present invention describes a postbiotic composition that comprises lysates of probiotic microorganisms as immunomodulatory agents and modulators of angiogenesis, which is extremely safe, including with a long period of consumption. Such a composition can be used as a food supplement.

As such, the present invention is included in the technical field of therapies whose purpose it is to contribute to the effect of cancer treatments, such as chemotherapy, radiotherapy, or immunotherapy.

## BACKGROUND

Cancer is one of the main causes of morbidity and mortality in the world. That is why in recent decades countless resources have been invested to develop therapies that are effective against this disease. These are therapies such as radiotherapy, chemotherapy, or immunotherapy combined with surgery when this cancer is associated with the presence of a tumor mass, when the specialist deems it operable.

However, it is well known that, although these therapies are usually effective in most cases, the side effects have a high toxicity on the patient's body. This generates a deterioration of their general state of health that calls into question the suitability of these therapies, whilst serving as an incentive for specialists to seek alternative treatments.

In general, alternative cancer treatments do not play a direct role in curing cancer, but they can help deal with the signs and symptoms of cancer and cancer treatments, such as anxiety, tiredness, nausea and vomiting, pain, difficulty sleeping, and stress.

Alternative medicine is a term commonly used to describe methods not typically offered by health care providers. As researchers study these treatments and evidence for these alternative methods increases, doctors and other providers are including them in treatment plans alongside standard treatments. It is an approach that specialists sometimes call integrative medicine.

Using these evidence-based integrative medicine approaches in conjunction with standard treatments can help alleviate many of the symptoms associated with cancer and its treatment, however, alternative or integrative treatments are often not effective enough to completely replace standard treatments.

That is why science constantly makes multiple efforts to find possible options to conventional cancer treatments that are not harmful to the patient's body, but that are effective in eradicating this disease.

There are publications that have developed different aspects on the dynamism of the intestinal microbiota and its variations due to the passing of time and to diseases, as well as other factors that can alter it and contribute to an improvement in the general condition of the patient.

Increasingly well-founded evidence supports not only the functional role of the intestinal microbiota in the development of cancer and its progression, but also in defining a significant role in the efficacy and toxicity of chemotherapeutic agents and immunotherapeutic compounds. Therapeutic oncological interventions may see their response vary according to the state of the microbiota. A loss of diversity in the gut microbiota has a direct impact on the efficacy and toxicity of chemotherapy, and can come indirectly from immune regulation.

Postbiotics described as a collection of molecules including nonfunctional microbial cells, cell compartments, cellular metabolites and metabolic bioproducts of probiotics, when consumed in sufficient quantities, can influence physiological processes, and further retain the ability to modulate the gut microbiota to maintain its homeostasis.

Accordingly, faced with the technical problem posed by current oncological treatments, the sector is actively working to develop treatments and compositions that can be administered orally to improve the effectiveness of cancer treatments. As proof of this, the following patent documents are cited.

Patent application number CN114847483A provides a composition comprising *Bifidobacterium longum* BL21 and a microbial inoculum containing *Bifidobacterium longum* BL21 in the preparation of products to prevent, alleviate or treat colorectal cancer.

Patent application CN102475776A discloses a cancer inhibitor, which is a new product among currently invented products used to control various cancers. The product is prepared from various materials of mineral selenium, magnesium, zinc, vitamin E, vitamin C, garlicin, probiotics, and pimpernel root powder.

International patent application WO2019169179A1, describing a method to improve the efficacy of a cancer therapy comprising formulations composed of probiotics and digestive enzymes.

However, none of the documents found in the prior art disclose a stable composition that has an effective immunomodulatory and modulating angiogenesis activity to improve the quality of life of patients with solid tumors through oral ingestion, such as that described in this document, that is composed of dry bacterial lysates.

It is also worth mentioning the Spanish patent applications P201930242 and P201930280, patent applications of the inventors that describe a modulating composition of the human intestinal microbiome obtained from lysates of

2

probiotic microorganisms and their method of obtainment, as well as a food supplement that includes the composition, which is useful in the prevention and treatment of disorders caused, or at least fostered, by the intestinal dysbiosis of the microbiota in humans. However, these applications do not disclose the specific composition of the present invention or its effect on cancer patients. An skilled person appreciates that, although in the prior art, there are many compositions with anti-inflammatory capacity demonstrated in in-vitro experiments, these results are not easily reproducible in humans. A quick search in databases can yield a multitude of results in vitro, but not in humans, because when consequently testing its effectiveness, it is non-existent. These negative results are not published. The recent article by Lu K, Dong S, Wu X, Jin R and Chen H (2021) Probiotics in Cancer. Front. Oncol. 11:638148. DOI: 10.3389/FONC.2021.638148 mentions these problems, as well as the need for further research to determine the specific composition of microorganisms that is most effective. (See the conclusions section of said article).

Therefore, the present invention aims to solve the problems of the state of the art by using a postbiotic composition of oral administration obtained from lysates of probiotic microorganisms for use as an immunomodulator and angiogenesis modulator for the improvement of state of health in patients with solid tumors.

## BRIEF DESCRIPTION OF THE INVENTION

In the present report the expressions "composition of oral administration", "composition of the invention" and "postbiotic composition" are synonymous and are used interchangeably.

In the present report, the term microorganisms includes both bacteria and yeast.

Immunomodulatory action is understood, in the context of the present invention, as the set of molecules of the cell walls and lysate of cells and non-viable cells that modulate the innate immune response by the release of cytokines and thereby enhances the immune response against cancer.

Modulating activity of angiogenesis is understood, in the context of the present invention, as the formation of new capillaries generated by lysates during embryonic development.

In a first aspect, the present invention relates to a postbiotic composition of oral administration for use in the treatment of solid tumors, and is characterized in that it comprises lysates of probiotic microorganisms in a percentage amount by weight with respect to the total weight of the lysates of microorganisms of the composition:

- 6% to 19% of bacterial lysates of the genus *Bacillus;*

- 4% to 8% of bacterial lysates of the genus *Bifidobacterium;*

- 15% to 25% of bacterial lysates of the genus *Lactobacillus;*

- 50% to 60% of yeast lysates of the genus *Saccharomyces;*

- 1.5% to 5% of bacterial lysates of the genus *Streptococcus.*

Another additional object of the invention relates to a pharmaceutical composition comprising an effective pharmaceutical amount of the composition in accordance with the first aspect of the invention and a pharmaceutically acceptable excipient.

## BRIEF DESCRIPTION OF THE FIGURES

To complement the description and help to gain a better understanding of the invention, we have included as an integral part of said description, a set of figures where, for illustrative and nonlimiting purposes, we present graphs of the experimental results obtained in examples X to Y that are collected in this document.

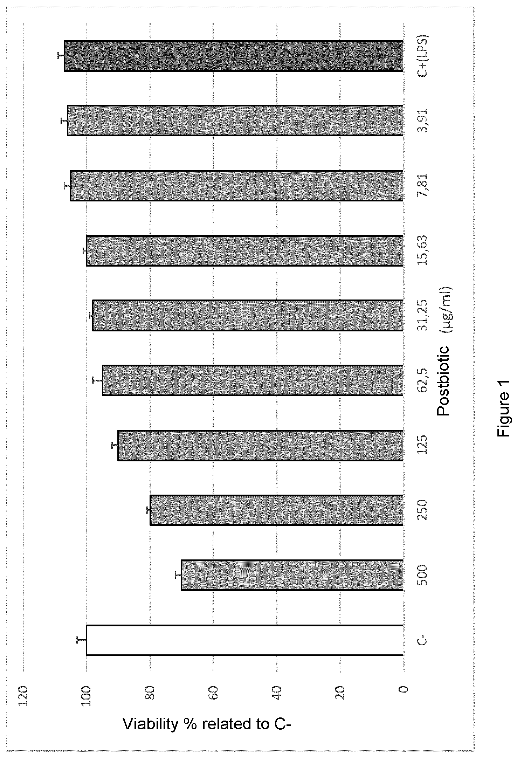

Figure 1. Percentage of cell viability after 24 hours of exposure to different concentrations of the postbiotic composition of the invention under study by MTT assay in THP-1 cell lines differentiated with PMA. The bars represent the average of six technical repetitions and the error bars correspond to the standard deviation. Negative control (C-) represents a condition in which cells were not exposed to any compound. The positive control (C+) represents a condition in which the cells were exposed to an inflammatory stimulus of LPS (Lipopolysaccharide). The selected concentrations are 15.63, 7.81 and 3.91 $\mu$g/ml since they do not affect cell viability. The term "postbiotic" is the postbiotic composition of the invention.

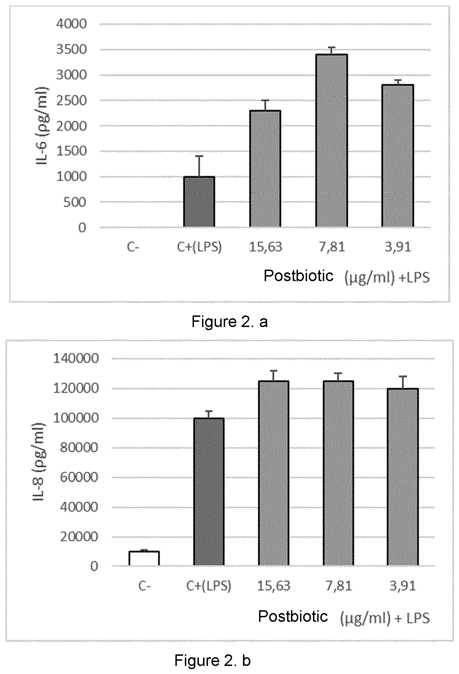

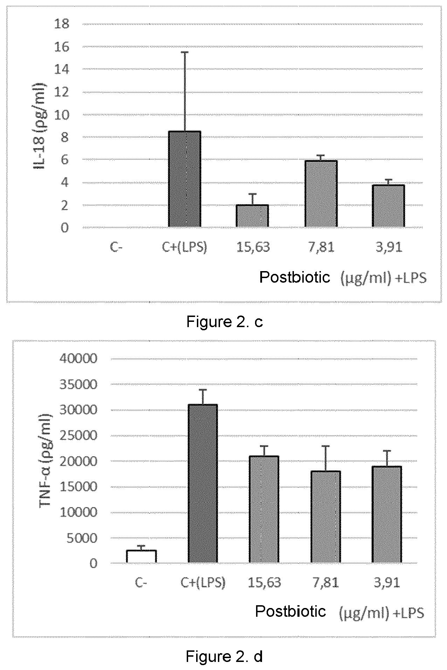

Figure 2. Secretion of proteins IL-6 (Figure 2.a), IL-8 (Figure 2.b), IL-18 (Figure 2.c) and TNF-$\alpha$ (Figure 2.d) by THP-1 cells stimulated with LPS under different concentrations of the postbiotic composition of the invention. The bars represent the average of three replicas and the error bars correspond to the standard deviation. Negative control (C-) represents a condition in which cells were not exposed to any compound. Positive control (C+) represents a condition in which cells were exposed to an inflammatory stimulus of LPS. The term "postbiotic" is the postbiotic composition of the invention.

**Figure 3.** Representative pictures of control embryos and treated at 48 hpf (hours post-fertilization) with the postbiotic composition of the invention (IGEN-1806).

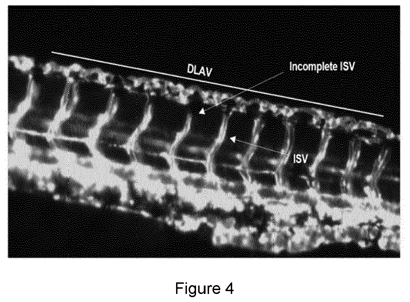

**Figure 4.** Fluorescent picture showing DLAV (dorsal longitudinal anastomotic vessel) and ISV (intersegmental blood vessels) positions.

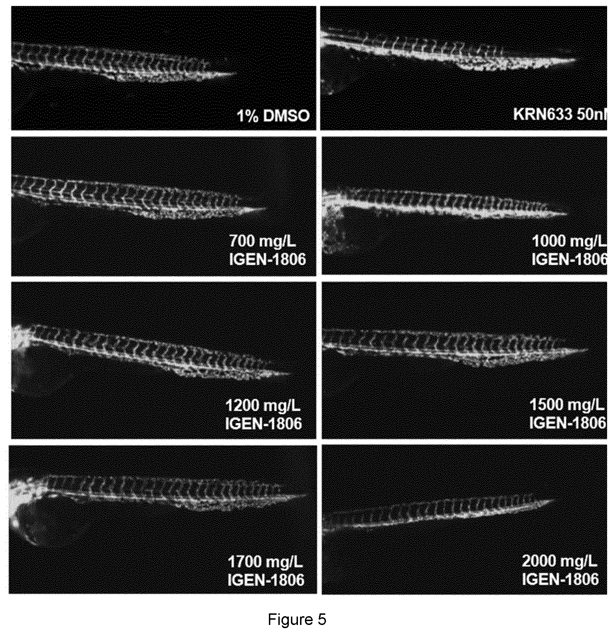

**Figure 5.** Representative fluorescent pictures showing the vasculature of untreated embryos at 48 hpf (DMSO, 1% dimethyl sulfoxide), positive control (KRN633, selective vascular endothelial growth factor receptor inhibitor) and embryos exposed to the postbiotic composition of the invention (IGEN-1806) at the indicated concentrations.

**Figure 6.** Representative fluorescent pictures showing the vasculature of untreated embryos at 72 hpf (DMSO 1 %), positive control (KRN633) and embryos exposed to the postbiotic composition of the invention (IGEN-1806) at the indicated concentrations.

## DETAILED DESCRIPTION OF THE INVENTION

As indicated in the previous sections, the inventors have detected the need to develop a postbiotic composition for oral administration that can be administered as a food supplement to improve the state of health of patients with a solid tumor, which is easy to administer and that does not present toxicity, and that also increases the effect of chemotherapy, radiation therapy or immunotherapy.

The terms "treatment" and "treat" refer herein to the medical treatment of a subject suffering from cancer with the intention of curing, improving, stabilizing, or preventing an aggravation of the disease, pathological condition or disorder. This term includes active treatment, that is, treatment aimed specifically at the improvement of said disease, pathological condition or disorder. In addition, this term includes palliative treatment, i.e. treatment designed to relieve symptoms rather than cure the disease, pathological condition or disorder; preventive treatment, i.e. treatment aimed at minimizing or partially or completely inhibiting the development of the associated disease, pathological condition or disorder; and supportive treatment, i.e. treatment used to complement other specific therapy aimed at improving the associated disease, pathological condition or disorder. It is understood that treatment, although intended to cure, improve, stabilize, or prevent a disease, pathological condition or disorder, does not have to actually produce cure, improvement, stabilization or prevention. The effects of treatment may be measured or evaluated as described in this document and as known in the prior art, as appropriate for the disease, pathological condition or disorder in question. Such measurements and evaluations can be made in qualitative and/or quantitative terms. Thus, for example, the characteristics or traits of a disease, pathological condition or disorder and/or the symptoms of a disease, pathological condition or disorder can be reduced to any effect or in any quantity. In the context of a subject suffering from cancer, the terms "treatment" and "treat" refer to the medical management of a subject with the intention of curing, improving or stabilizing the cancer, as well as improving the symptoms associated with this disease. Based on this, a composition that is administered orally has been developed, comprising lysates of microorganisms that can be accompanied by other components for this composition, and that has turned out to be surprisingly beneficial for humans and animals since it presents an immunomodulatory and angiogenesis modulating action .

In the context of the present invention, what is understood by immunomodulatory action is a postbiotic composed of a set of molecules from the lysate of cells and non-viable cells that has the ability to modulate the innate immune response. The inventors have been able to verify that the fact of administering the present composition described in the document, has the ability to regulate pro-inflammatory mediators with the capability of acting against infections and cancer cells. The latter is shown in example 1. In the context of the present invention, what is understood by angiogenesis modulator is a lysate that has the property of inducing the formation of new capillaries during embryonic development, that is, proangiogenesis. The inventors have been able to verify that the fact of administering the present composition described in the document, has the ability to induce the formation of new vessels only in the early stages of formation of the zebrafish embryo, improving the bioavailability of nutrients, as shown in example 2.

Usually, probiotics when consumed, can positively influence the composition of the intestinal microbiota and interact with different immune cells, thus improving immune functions. It is widely accepted that their administration brings health benefits. In a hostile intestinal environment such as that of a cancer patient, however, they usually have difficulties to survive due to the presence of growth conditions that are not optimal. The administration of cytotoxic agents induces changes in the structure and functions of the intestinal microbiota. These reasons may mean that the use of probiotics does not present strong results to observe a positive effect on health. However, the composition, object of the invention, stands out for its immediate and surprising effect, not only on tumor masses (see example 3), but in the general state of the patient.

Probiotics are live microorganisms that, once they colonize the gastrointestinal tract, provide a health benefit due to their own physiological activity. This requires surviving gastric juices, digestive enzymes, and bile acids, so it is usual to add them in a sufficient amount for the effect to be evident. On the other hand, postbiotics do not require this activity as they are inanimate microorganisms and/or their components, thus presenting a technological advantage.

Postbiotics have the potential to directly modulate the composition of the intestinal microbiota as well as indirectly mediate the effectiveness of cancer treatment through immune protection to the host through the regulation, stimulation, and modulation of the immune response. They provide a safe alternative to probiotics.

Thus, in the first aspect, the present invention relates to a postbiotic composition for oral administration for use in the treatment of solid tumors, characterized in that it comprises lysates of probiotic microorganisms in an amount in percentage by weight with respect to the total weight of the lysates of microorganisms of the composition:

- 6% to 19% of bacterial lysates of the genus *Bacillus;*

- 4% to 8% of bacterial lysates of the genus *Bifidobacterium;*

- 15% to 25% of bacterial lysates of the genus *Lactobacillus;*

- 50% to 60% of yeast lysates of the genus *Saccharomyces;*

- 1.5% to 5% of bacterial lysates of the genus *Streptococcus.*

In a particular embodiment, the postbiotic composition of oral administration for use in the treatment of solid tumors comprises lysates of probiotic microorganisms in an amount in percentage by weight with respect to the total weight of lysates of microorganisms of the composition:

- 13% to 19% of bacterial lysates of the genus *Bacillus;*

- 4% to 8% of bacterial lysates of the genus *Bifidobacterium;*

- 15% to 25% of bacterial lysates of the genus *Lactobacillus;*

- 50% to 60% of yeast lysates of the genus *Saccharomyces;*

- 1.5% to 5% of bacterial lysates of the genus *Streptococcus.*

In accordance with the invention, the composition can comprise between 1% - 99.5% by weight of lysates of microorganisms; particularly 5%-99% by weight of lysates of microorganisms, particularly 10%-96% by weight of lysates of microorganisms, particularly 25%-95% by weight of lysates of microorganisms, or particularly 50%±10% by weight of lysates of microorganisms.

The composition of the invention may comprise additional components or additives, or any other substance, such as salts or excipients that facilitate packaging, preparation and/or administration of the composition, but do not affect the immunomodulating or angiogenesis-promoting capacity of the postbiotic composition of the invention.

In the context of the present invention, lysates of probiotic microorganisms are understood to be the product obtained after the process of cultivating, and subsequently, mechanically or chemically breaking said cells in order to obtain a product with microbial fragments, as well as all the components that are comprised within. Likewise, it is indicated in this document that these are lysates of dry microorganisms, since, thanks to their method of obtainment, these lysates are subsequently subjected to drying techniques, in such a way that said dry bacterial lysates are optionally in the form of dry powder.

In preferred embodiments of the present invention, the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus, Bacillus paralicheniformes,* and combinations thereof. Preferably, *Bacillus clausii, Bacillus coagulans, Bacillus licheniformis, Bacillus mesentericus* and *Bacillus subtilis.*

In other embodiments, the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof, preferably *Bifidobacterium lactis.*

In other embodiments, the bacterial lysates of the genus *Lactobacillus* are of the species that are selected from the group consisting of *Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus, Lactobacillus salivarius, Lactobacillus helvéticus, Lactobacillus gasseri, Lactobacillus johnsonii, Lactobacillus brevis, Lactobacillus kefiri,* and combinations thereof, preferably *Lactobacillus acidophilus, Lactobacillus bulgaricus, Lactobacillus casei, Lactobacillus fermentum, Lactobacillus reuteri,* and *Lactobacillus rhamnosus.*

In other particular embodiments of the present invention, the lysates of yeasts of the genus *Saccharomyces* are of the species that are selected from the group consisting of *Saccharomyces cerevisiae, Saccharomyces boulardi,* and combinations thereof, preferably *Saccharomyces cerevisiae.*

In other particular embodiments of the present invention, the bacterial lysates of the genus *Streptococcus* are of the species *Streptococcus thermophilus.*

In a particular embodiment, the composition of the invention does not comprise lysates of microorganisms present in the digestive tract, preferably bacterial lysates of the genera *Akkermansia, Enterococcus* and *Escherichia.*

In preferred embodiments of the present invention, the composition of the invention comprises bacterial lysates of the genus *Bacillus* in an amount in percentage by weight between approximately 6 % to 19 % of the total lysates of the composition, preferably 13 % to 19 % of the total lysates of the composition. In preferred embodiments, the amount of bacterial lysates of the genus *Bacillus* can be approximately 14%, 15%, 16%, 17%, or 18% of the total lysates of the composition.

In a particular embodiment, the percentage by weight of *Bacillus licheniformis, Bacillus mesentericus* and *Bacillus subtilis* is approximately the same, and is higher than that of *Bacillus clausii* and *Bacillus coagulans,* which is also approximately the same.

In preferred embodiments of the present invention, the composition of the invention comprises bacterial lysates of the genus *Bifidobacterium* in an amount in percentage by weight between approximately 4% to 8% of the total lysates of the composition, preferably between 5% to 7%. In preferred embodiments, the amount of bacterial lysates of the genus *Bifidobacterium* can be approximately 5%, 6%, 7%, or 8% of the total lysates of the composition.

In preferred embodiments of the present invention, the composition of the invention comprises bacterial lysates of the genus *Lactobacillus* in an amount in percentage by weight between approximately 15% to 25% of the total lysates of the composition, preferably between 20% to 24% of the total lysates of the composition. In preferred embodiments, the amount of bacterial lysates of the genus *Lactobacillus* can be about 20%, 21%, 22%, 23%, 24%, or 25% of the total lysates of the composition.

In a particular embodiment, the percentage by weight of *Lactobacillus acidophilus, Lactobacillus bulgaricus* and *Lactobacillus casei* is different from each other and different from the percentage by weight of *Lactobacillus fermentum, Lactobacillus reuteri* and *Lactobacillus rhamnosus,* which is approximately identical between these three species.

In another particular embodiment, the percentage by weight of *Lactobacillus casei* is higher than the rest of *Lactobacillus* species.

In another particular embodiment, the percentage by weight of *Lactobacillus casei* is higher than that of *Lactobacillus acidophilus,* which in turn is higher than that of *Lactobacillus bulgaricus,* which in turn is higher than that of *Lactobacillus fermentum, Lactobacillus reuteri* and *Lactobacillus rhamnosus,* the percentage by weight of these 3 species being approximately identical.

In preferred embodiments of the present invention, the composition comprises lysates of yeasts of the genus *Saccharomyces* in an amount in percentage by weight of between approximately 50% to 60% of the total lysates of the composition, preferably between 52% to 57% of the total lysates of the composition. In preferred embodiments, the amount of lysates of yeasts of the genus *Saccharomyces* can be about 50%, 51%, 52%, 53%, 54%, 55%, 56%, 57%, 58%, 59%, or 60% of the total lysates of the composition.

In preferred embodiments of the present invention, the composition comprises bacterial lysates of the genus *Streptococcus* in an amount in percentage by weight of between about 1.5% to 5% of the total lysates of the composition, preferably between about 3% to 4% of the total lysates of the composition. In preferred developments, the amount of bacterial lysates of the genus *Streptococcus* can be about 1.5%, 1.6%, 1.7%, 1.8%, 1.9%, 2%, 2.1%, 2.2%, 2.3%, 2.4%, 2.5%, 2.6%, 2.7%, 2.8%, 2.9% or 3% of the total lysates of the composition. In other preferred embodiments, the amount of bacterial lysates of the genus *Streptococcus* can be about 3.5%, 4%, 4.5% or 5% of the total lysates of the composition.

In a particular embodiment of the invention, the composition comprises lysates of probiotic microorganisms in an amount in percentage by weight of the total lysates of the composition:

- approximately 13% to 17% of bacterial lysates of the genus *Bacillus;*

- approximately 4.5% to 7.5% of bacterial lysates of the genus *Bifidobacterium;*

- approximately 20% to 22% of bacterial lysates of the genus *Lactobacillus;*

- approximately 53% to 57% of yeast lysates of the genus *Saccharomyces;*

- approximately 2.5% to 3.5% of bacterial lysates of the genus *Streptococcus.*

This composition is called composition "A"

In another particular embodiment of the invention, the composition comprises lysates of probiotic microorganisms in an amount in percentage by weight of the total lysates of the composition:

- approximately 15% to 17% of bacterial lysates of the genus *Bacillus;*

- approximately 4.6% to 6% of bacterial lysates of the genus *Bifidobacterium;*

- approximately 20.5% to 21.8% of bacterial lysates of the genus *Lactobacillus;*

- approximately 54% to 56% of yeast lysates of the genus *Saccharomyces;*

- approximately 2.8% to 3.2% of bacterial lysates of the genus *Streptococcus.*

This composition is called composition "B"

In another particular embodiment of the invention, the composition comprises lysates of probiotic microorganisms in an amount in percentage by weight of the total lysates of the composition:

- approximately 6% to 19% of bacterial lysates of the genus *Bacillus;*

- approximately 4% to 8% of bacterial lysates of *Bifidobacterium lactis,* preferably approximately 4.5% to 7.5% of bacterial lysates of *Bifidobacterium lactis;*

- approximately 15% to 25% of bacterial lysates of the genus *Lactobacillus;*

- approximately 50% to 60% of yeast lysates of the genus *Saccharomyces;*

- approximately 1.5% to 5% of bacterial lysates of the genus *Streptococcus.*

This composition is called composition "C"

In another particular embodiment of the invention, the composition comprises lysates of probiotic microorganisms in an amount in percentage by weight of the total lysates of the composition:

- approximately 13% to 17% of bacterial lysates of the genus *Bacillus;*

- approximately 4% to 8% of bacterial lysates of *Bifidobacterium lactis,* preferably approximately 4.5% to 7.5% of bacterial lysates of *Bifidobacterium lactis;*

- approximately 20% to 22% of bacterial lysates of the genus *Lactobacillus;*

- approximately 53% to 57% of yeast lysates of the genus *Saccharomyces;*

- approximately 2.5% to 3.5% of bacterial lysates of the genus *Streptococcus.*

This composition is called composition "D"

In another particular embodiment of the invention, the composition comprises lysates of probiotic microorganisms in an amount in percentage by weight of the total lysates of the composition:

- approximately 15% to 17% of bacterial lysates of the genus *Bacillus;*

- approximately 4.6% to 6% of bacterial lysates of *Bifidobacterium lactis;*

- approximately 20.5% to 21.8% of bacterial lysates of the genus *Lactobacillus;*

- approximately 54% to 56% of yeast lysates of the genus *Saccharomyces*;

- approximately 2.8% to 3.2% of bacterial lysates of the genus *Streptococcus*.

This composition is called composition "E"

In another particular embodiment of the invention, the composition comprises lysates of probiotic microorganisms in an amount in percentage by weight of the total lysates of the composition:

- approximately 6% to 19% of bacterial lysates of *Bacillus clausii, Bacillus coagulans, Bacillus licheniformis, Bacillus subtilis* and *Bacillus mesentericus;*

- approximately 4% to 8% of bacterial lysates of *Bifidobacterium lactis;*

- approximately 15% to 25% bacterial lysates of *Lactobacillus acidophilus, Lactobacillus bulgaricus, Lactobacillus casei, Lactobacillus fermentum, Lactobacillus reuteri* and *Lactobacillus rhamnosus;*

- approximately 50% to 60% of yeast lysates of *Saccharomyces cerevisiae;*

- approximately 1.5% to 5% of bacterial lysates of *Streptococcus thermophilus.*

This composition is called composition "F"

In another particular embodiment of the invention, the composition comprises lysates of probiotic microorganisms in an amount in percentage by weight of the total lysates of the composition:

- approximately 13% to 17% of bacterial lysates of *Bacillus clausii, Bacillus coagulans, Bacillus licheniformis, Bacillus subtilis* and *Bacillus mesentericus;*

- approximately 4.5% to 7.5% of bacterial lysates of *Bifidobacterium lactis;*

- approximately 20% to 22% of bacterial lysates of *Lactobacillus acidophilus, Lactobacillus bulgaricus, Lactobacillus casei, Lactobacillus fermentum, Lactobacillus reuteri* and *Lactobacillus rhamnosus;*

- approximately 53% to 57% of yeast lysates of *Saccharomyces cerevisiae;*

- approximately 2.5% to 3.5% of bacterial lysates of *Streptococcus thermophilus.*

This composition is called composition "G"

In another particular embodiment of the invention, the composition comprises lysates of probiotic microorganisms in an amount in percentage by weight of the total lysates of the composition:

- approximately 15% to 17% of bacterial lysates of *Bacillus clausii, Bacillus coagulans, Bacillus licheniformis, Bacillus subtilis* and *Bacillus mesentericus;*

- approximately 4.6% to 6% of bacterial lysates of *Bifidobacterium lactis;*

- approximately 20.5% to 21.8% of bacterial lysates of *Lactobacillus acidophilus, Lactobacillus bulgaricus, Lactobacillus casei, Lactobacillus fermentum, Lactobacillus reuteri* and *Lactobacillus rhamnosus;*

- approximately 54% to 56% yeast lysates of *Saccharomyces cerevisiae;*

- approximately 2.8% to 3.2% of bacterial lysates of *Streptococcus thermophilus.*

This composition is called composition "H"

In another particular embodiment of the invention, the composition comprises lysates of probiotic microorganisms in an amount in percentage by weight with respect to the total:

- approximately 6% to 19% of bacterial lysates of *Bacillus licheniformis, Bacillus pumilus,* and *Bacillus subtilis,* the percentage by weight of *Bacillus licheniformis, Bacillus pumilus,* and *Bacillus subtilis* being approximately the same;

- approximately 4% to 8% of bacterial lysates of *Bifidobacterium lactis;*

- approximately 15% to 25% of bacterial lysates of *Lactobacillus acidophilus, Lactobacillus casei, Lactobacillus fermentum Lactobacillus reuteri, Lactobacillus rhamnosus* and *Lactobacillus bulgaricus,* the percentage by weight of *Lactobacillus casei* being higher than that of *Lactobacillus acidophilus,* which in turn is superior to that of *Lactobacillus bulgaricus,* which in turn is superior to that of *Lactobacillus fermentum, Lactobacillus reuteri* and *Lactobacillus rhamnosus,* the percentage by weight of these 3 strains being approximately identical;

- approximately 50% to 60% of yeast lysates of *Saccharomyces cerevisiae;*

- approximately 1.5% to 5% of bacterial lysates of *Streptococcus thermophilus.*

This composition is called composition "I"

In another particular embodiment of the invention, the composition comprises lysates of probiotic microorganisms in an amount in percentage by weight with respect to the total:

- approximately 13% to 17% of bacterial lysates of *Bacillus licheniformis, Bacillus pumilus* and *Bacillus subtilis,* the percentage by weight of *Bacillus licheniformis, Bacillus pumilus* and *Bacillus subtilis* being approximately the same;

- approximately 4.5% to 7.5% of bacterial lysates of *Bifidobacterium lactis;*

- approximately 20% to 22% of bacterial lysates of *Lactobacillus acidophilus, Lactobacillus casei, Lactobacillus fermentum Lactobacillus reuteri, Lactobacillus rhamnosus* and *Lactobacillus bulgaricus,* the percentage by weight of *Lactobacillus casei* being higher than that of *Lactobacillus acidophilus,* which in turn is superior to that of *Lactobacillus bulgaricus,* which in turn is superior to that of *Lactobacillus fermentum Lactobacillus reuteri* and *Lactobacillus rhamnosus,* the percentage by weight of these 3 strains being approximately identical;

- approximately 53% to 57% of yeast lysates of *Saccharomyces cerevisiae;*

- approximately 2.5% to 3.5% of bacterial lysates of *Streptococcus thermophilus.*

This composition is called composition "J"

In another particular embodiment of the invention, the composition comprises lysates of probiotic microorganisms in an amount in percentage by weight with respect to the total:

- approximately 15% to 17% of bacterial lysates of *Bacillus licheniformis, Bacillus pumilus* and *Bacillus subtilis,* the percentage by weight of *Bacillus licheniformis, Bacillus pumilus* and *Bacillus subtilis* being approximately the same;

- approximately 4.6% to 6% of bacterial lysates of *Bifidobacterium lactis;*

- approximately 20.5% to 21.8% of bacterial lysates of *Lactobacillus acidophilus, Lactobacillus casei, Lactobacillus fermentum Lactobacillus reuteri, Lactobacillus rhamnosus* and *Lactobacillus bulgaricus,* the percentage by weight of *Lactobacillus casei* being higher than that of *Lactobacillus acidophilus,* which in turn is superior to that of *Lactobacillus bulgaricus,* which in turn is superior to that of *Lactobacillus fermentum Lactobacillus reuteri* and *Lactobacillus rhamnosus,* the percentage by weight of these 3 strains being approximately identical;

- approximately 54% to 56% of yeast lysates of *Saccharomyces cerevisiae;*

- approximately 2.8% to 3.2% of bacterial lysates of *Streptococcus thermophilus.*

This composition is called composition "K"

Below, the quantities in which each of the additional components or additives can be included to optionally make up the composition according to the first aspect of the present invention will be indicated.

In other particular embodiments of the present invention, additional components or additives may be: carbohydrates (fructose, xylitol, sorbitol, fructooligosaccharides, inulin), antioxidants (resveratrol, beta-carotene), vitamins (Vitamin C, D, K, B1, B9, E, B3, B5, A, B3 , B6, H, D3, B12 biotin, riboflavin, pyridoxine, pantothenic acid), prebiotics (galacto-oligosaccharides, inulin, oligofructose), amino acids (cysteine, tyrosine, resin, methionine, phenylalanine, glycine, glutamine, alanine, carnitine, glutamine, arginine), lipids (eicosapentaenoic acid, docosahexaenoic acid, arachidonic acid), trace elements (sodium, potassium, magnesium, phosphorus, calcium, copper, zinc, manganese, chromium, iodine, selenium), digestive enzymes (papain, amylase, lactase, bromelain), whey.

These micro and macronutrients can modulate the intestinal microbiota and favour the predominance of some species associated with a good state of health, such as species of the genera *Bifidobacterium, Lactobacillus, Akkermansia, Fecalibacterium, Eubacterium, Roseburia, Ruminococcus* and *Blautia.* Among the macronutrients, carbohydrates such as galactooligosaccharides, fructooligosaccharides and inulin have a bifidogenic effect and can modulate microorganisms beneficial to health as well as unsaturated fat, which also increases the beneficial microbiota. Antioxidants such as polyphenols modulate the Firmicutes/Bacteroideratio ratio. Vitamins, such as vitamin A, C, D and E have a positive influence on *Bifidobacteria, Akkermansia* and *Lactobacillus.* Some trace elements such as calcium, magnesium, phosphorus, selenium and zinc have shown an effect on *Akkermansia, Bifidobacterium* and *Ruminococcus.* Prebiotic consumption has been associated with the growth of *Bifidobacterium* and lactic acid bacteria. In general, macro and micronutrients influence the composition and diversity of the gut microbiota.

In a particular embodiment of the invention, the composition of lysates and additional components is that of table 1. Additional components may be one or more of resveratrol, astaxanthin, bromelain, papain, fermented rice starch, corn starch and FOS, each of these components in varying amounts, as shown in Table 1.

Table 1: List of additional components to the composition of the invention per 100 mg of lysates of probiotic microorganisms.

| Composition of lysates of probiotic microorganisms | 100 | Mg |
|---|---|---|
| Resveratrol | Up to 2.55 | Mg |
| Astaxanthin | Up to 25 | Mg |
| Bromelain | Up to 24 | Mg |
| Papain | Up to 35.2 | Mg |
| Fermented rice starch | Up to 110 | Mg |
| Corn starch | Up to 12 | Mg |
| FOS | Up to 70 | Mg |

In the specific example of Table 1, the composition of lysates of probiotic microorganisms would represent 26.4% of the total weight of the composition of the invention.

In particular embodiments of the present invention, the composition of the invention, is obtained by a method comprising the following steps:

- Cultivate the microbial species selected for the preparation of the composition using the standard conditions established for the microbial species listed in this document;
- Filter or centrifuge the microbial species until a suitable biomass of the selected microorganisms is obtained;
- Lyse through freezing and thawing and sonication cycles of the biomasses obtained. until securing at least 90% or 91% or 92% or 94% of bacterial lysates, preferably at least 95%, or 96% or 97% or 98% and more preferably, 99% or 100%;
- Mix the different strains in the proportions selected according to the percentage by weight to obtain the composition of lysates of probiotic microorganisms of the invention.

A skilled person would know how to find the appropriate protocol for the growth of the microorganisms of the composition, as well as the protocol for their lysis, since these are common procedures in the prior art.

In a particular embodiment, the method of obtaining the composition of the invention comprises an additional step: drying by atomization or lyophilization the biomasses of bacterial lysates obtaining a product in powder form comprising all the bacterial strains selected to prepare the composition object of the invention.

In a particular embodiment, the composition of the invention is presented in the form of dry powder.

When the composition of the invention is presented in the form of dry powder, it is necessary to dissolve it in water or any other liquid that does not affect the properties of the components of the composition prior to its intake by the patient. The advantages of dry powder are that it is easier to transport and to store, and it also increases the stability and durability of the composition. In the present document, dry powder means that the humidity level is less than 10%, preferably less than 5%, even more preferably less than 1%.

In particular embodiments, the composition of the invention is obtained from cultures of probiotic microorganisms of the genera described in the present document, which comprise a certain amount of Colony Forming Units (CFU). In the context of the present invention, a Colony Forming Unit is a term of microbiology. It is an indicator of the amount of live microorganisms present in a medium.

The process of obtaining lysates comprises a thermal treatment, alternating heating and cooling cycles. Each batch of viable cell culture is resuspended in distilled water in a 1:2 ratio and subjected to a sterilization cycle in an autoclave at 121°C for 20 minutes. They are then frozen at -20°C for 12 hours and thawed at room temperature the next day.

Once thawed, they undergo a cell rupture treatment by sonication for 20min, at an output power of 500 W at an amplitude of 40% and 10 seconds of pause, obtaining a mass of lysed cells from the probiotic batch (Qsonica, Q500). The final solution is freeze-dried and ground to obtain a lysate of the powdery probiotic microorganism. The powder is kept in a cool environment away from heat. The degree of survival in no case exceeds a final viability of approximately 7 E3 CFU/ml, which implies practically 100% cell death.

In particular embodiments of the present invention, one starts with a quantity of bacteria of the genus *Bacillus* comprising between 3.49% to 20.94% of CFU with respect to the total CFU of the composition, preferably between 10.98% and 15.75%. In absolute values, the culture grown, before proceeding to the lysis of the cells, presents between 1E+11 and 5E+11 cells, preferably between 2E+11 and 3E+11, most preferably approximately 2.86E+11.

In another particular embodiment, when the species of the genus *Bacillus* are: *Bacillus clausii, Bacillus coagulans, Bacillus licheniformis, Bacillus mesentericus* and *Bacillus subtilis,* each of the grown cultures presents, before proceeding to the lysis of the cells, between 3E+9 and 9E+9 *Bacillus clausii* cells, between 3E+9 and 9E+9 of *Bacillus coagulans* cells, between 9E+10 and 3E+11 of *Bacillus licheniformis* cells, between 6E+10 and 9E+10 of *Bacillus mesentericusy* cells and between 9E+10 and 3E+11 of *Bacillus subtilis* cells.

In particular embodiments of the present invention, one starts with a number of bacteria of the genus *Lactobacillus* comprising between 28.64% to 66.82% of CFU with respect to the total CFU of the composition, preferably between 40.66% to 57.36%. In absolute values, the culture grown, before proceeding to the lysis of the cells, presents between 8E+11 and 3E+12 cells, preferably between 9E+11 and 2E+12, most preferably approximately 1.07E+12.

In another particular embodiment, when the species of the genus *Lactobacillus* are: *Lactobacillus acidophilus, Lactobacillus bulgaricus, Lactobacillus casei, Lactobacillus fermentum, Lactobacillus reuteri* and *Lactobacillus rhamnosus,* each of the grown cultures presents, before proceeding to the lysis of the cells, between 5E+10 and 9E+10 cells of *Lactobacillus acidophilus,* between 5E+10 and 9E+10 cells of *Lactobacillus bulgaricus,* between 2E+11 and 8E+11 cells of *Lactobacillus casei,* between 3E+10 and 9E+10 cells of *Lactobacillus fermentum,* between 3E+10 and 9E+10 cells of *Lactobacillus reuteri* and between 1E+11 and 6E+11 cells of *Lactobacillus rhamnosus.*

In particular embodiments of the present invention, one starts with a number of bacteria of the genus *Streptococcus* comprising between 5.72% to 38.15% of CFU with respect to the total CFU of the composition, preferably between 10.98% to 20.75%. In absolute values, the culture grown, before proceeding to the lysis of the cells, presents between 1E+11 and 6E+11 cells, preferably between 2E+11 and 4E+11, most preferably approximately 3.00E+11. In a particular embodiment, the species of *Streptococcus* is *Streptococcus thermophilus.*

In particular embodiments of the present invention, one starts with a quantity of bacteria of the genus *Saccharomyces* comprising between 11.44% to 24.79% of CFU with respect to the total culture, preferably between 19.04% to 21.75%. In absolute values, the culture grown, before proceeding to the lysis of the cells, presents between 1 E+11 and 7E+11 cells, preferably between 4E+11 and 6E+11, most preferably approximately 5,20E+11. In a particular embodiment, the species of *Saccharomyces* is *Saccharomyces cerevisie.*

In particular embodiments of the present invention, one starts with a quantity of bacteria of the genus *Bifidobacterium* comprising between 10.20% to 53.64% of CFU with respect to the total culture, preferably between 15.99% to 18.75%. In absolute values, the culture grown, before proceeding to the lysis of the cells, presents between 1 E+11 and 7E+11 cells, preferably between 4E+11 and 6E+11, most preferably approximately 4.50E+11. In a particular embodiment, the species of Bifidobacterium is *Bifidobacterium lactis.*

These probiotic bacteria are cultured under standard conditions, as set out in the culture protocols published by the Spanish Collection of Type Cultures (CECT), indicated for each of the bacterial species described in this document.

Once these microorganisms are cultured, they are subjected to a lysis procedure. First of all, the microbial cells undergo heat treatment. Each batch of viable cell culture undergoes a sterilization cycle in an autoclave at 121°C for 20 to 30 minutes. This temperature denatures and coagulates the proteins by inactivating them. In addition, it causes damage to membranes, aggregation of ribosomes, breaking of DNA strands and inactivation of enzymes. Once cold, they undergo a cell rupture treatment by sonication for a period of 15 to 20min, at an output power of between 450 to 550 W, at an amplitude of between 38 to 43% and a period of 8 to 15 seconds of pause, obtaining a mass of lysed cells from the probiotic batch (Qsonica, Q500). This achieves the breakage of intermolecular interactions and DNA fragmentation. Optionally, the final solution is freeze-dried and ground to obtain a lysate of the powdery probiotic microorganism. The powder is kept in a cool environment away from heat.

The composition, in accordance with the invention, is the result of the combination of probiotic microorganisms that, after a process of growth and lysis, generates an extract consisting of a set of metabolites, proteins, DNA fragments and other components, such as, for example, peptidoglycans, which through efficient dosing is able to alter and modify in a surprising way the microbiota of the host through several mechanisms, so as to confer an activation of the immune system, reversing dysbiosis and surprisingly enhancing the delay of cellular aging.

In a particular embodiment, when the composition of the invention is in powder form, the composition, in line with the first aspect of the invention, can be presented in sealed sachets, which is in itself conventional in the food and pharmaceutical industry.

Another form of presentation of the food supplement, in accordance with the invention, is in capsule form, such as conventional gelatin capsules, inside which the composition in powder form is contained.

In another particular embodiment, the treatment of solid tumors comprises the improvement of patients' quality of life through the immunomodulatory and modulating properties of angiogenesis of the composition of the invention.

In an additional embodiment, improvement of quality of life concerns one or more of the following symptoms: anxiety, tiredness, nausea, vomiting, pain, difficulty sleeping, stress, asthenia, appetite, pain, dyspnea and diarrhea.

In the present invention solid tumors relate to one or more of the following: prostate cancer, breast cancer, cancer of the central nervous system (CNS), pancreatic cancer, esophageal cancer, uterine cancer, lymphoma, pheochromocytoma and sarcoma.

In a preferred embodiment, solid tumors are prostate, CNS, esophagus, pheochromocytoma and sarcoma.

In the present invention, solid tumors do not include colon cancer or lung cancer, nor subtypes of such tumors. These tumors are not under the scope of protection of the present invention.

In a particular embodiment, the patient is a terminal cancer patient. In the present document, a patient with terminal cancer is one who, before receiving the composition of the invention, receives palliative care or medication to improve their quality of life, but not treatments to try to cure the cancer, since these have proved inefficient.

An additional advantage of the invention is that the treatment with the composition of the invention allows better toleration of chemotherapy, radiotherapy and immunotherapeutic treatments, even enabling the re-administration of treatments that had been abandoned in patients due to toxicity.

Thus, in the context of the present invention, it is established that at least one dose must be administered, preferably at least one daily dose, between 100mg to 80000mg of the composition of the invention, including any of the compositions A-K. In preferred embodiments, a dosage may comprise between 100mg to 50000mg of the composition of the invention, including any of the compositions A-K; in another preferred embodiment, a dosage may comprise between 100mg to 2000mg of the composition of the invention, including any of the compositions A-K; in another preferred embodiment, a dosage may comprise between 100mg to 700mg of the composition of the invention, including any of the compositions A-K. In particular embodiments of the present invention, a dosage may comprise approximately 120mg, 130mg, 140mg, 150mg, 160mg, 170mg, 180mg, 190mg, 200mg 210mg, 220mg, 230mg, 240mg, 250mg, 260mg, 270mg, 280mg, 290mg, 300mg 310mg, 320mg, 330mg, 340mg, 350mg, 360mg, 370mg, 380mg, 390mg, 400mg, 410mg, 420mg, 430mg, 440mg, 450mg, 460mg, 470mg, 480mg, 490mg, 500mg, 510mg, 520mg, 530mg, 540mg, 550mg, 560mg, 570mg, 580mg, 590mg, 600mg, 610mg, 620mg, 630mg, 640mg, 650mg, 660mg, 670mg, 680mg, or 690mg of the composition of the invention, including any of the compositions A-K.

In a particular embodiment, you can administer at least one dose of the composition of the invention, including any of the compositions A-K, daily, or every other day, or every 3 or 4 days, following any of the concentrations of the preceding paragraph.

In another additional embodiment, the administration of the postbiotic composition of the invention, including any of the compositions A-K, is daily for at least 2 weeks, preferably daily for at least 3 weeks, even more preferably daily for at least 4 weeks, even more preferably daily for at least 5 weeks, even more preferably daily for at least 6 weeks, even more preferably daily for at least 7 weeks, even more preferably daily for at least 8 weeks, or daily for at least 9 weeks, or daily for at least 10 weeks, or daily for at least 11 weeks, or daily for at least 12 weeks.

Preferably the composition of the invention, including any of the compositions A-K, is administered in an amount between 100mg to 700mg per dose and between 1 to 6 doses per day for at least 2 weeks.

In another particular embodiment, the composition of the invention, including any of the compositions A-K, is administered in an amount between 150mg to 650mg per dose and between 1 to 5 doses per day for at least 3 weeks.

In another particular embodiment, the composition of the invention, including any of the compositions A-K, is administered in an amount between 200mg to 600mg per dose and between 2 to 4 doses per day for at least 4 weeks.

In another particular embodiment, the composition of the invention, including any of the compositions A-K, is administered in an amount between 250mg to 550mg per dose and between 2 to 4 doses per day for at least 5 weeks.

In another particular embodiment, the composition of the invention, including any of the compositions A-K, is administered in an amount between 300mg to 500mg per dose and between 2 to 4 doses per day for at least 6 weeks.

In another particular embodiment, the composition of the invention, including any of the compositions A-K, is administered in an amount between 300mg to 500mg per dose and between 2 to 4 doses per day for at least 7 weeks.

In another particular embodiment, the composition of the invention, including any of the compositions A-K, is administered in an amount between 300mg to 500mg per dose and between 2 to 4 doses per day for at least 8 weeks.

In another additional embodiment, the treatment of the postbiotic composition is carried out before, simultaneously or after the treatment of the patient with chemotherapy or radiotherapy or any other cancer treatment. When the treatment with the composition of the invention manages to improve the general condition of the patient, chemotherapy or radiotherapy or any other cancer treatment can be performed if the doctors consider that the patient can withstand them.

In another particular embodiment, the amount of the composition is different every time that it is administered to the patient during the day.

This beneficial effect is due to the fact that the composition of the invention can control the formation of blood vessels in solid tumors. Thus, an additional object of the invention relates to the composition of the invention for use as a proangiogenic agent.

In the context of the present invention, what is understood by proangiogenic is an agent that is used to promote the development of collateral blood vessels, capable of making up for perfusion deficiency secondary to obstruction of native arteries.

An additional object of the invention relates to a pharmaceutical composition comprising an effective pharmaceutical amount of the composition, in accordance with the first aspect of the invention, and a pharmaceutically acceptable excipient.

In the context of the present invention, the expression "pharmaceutical composition" refers to a formulation that has been adapted to deliver a predetermined dose of one or more useful therapeutic agents to a cell, a group of cells, an organ, or a tissue.

The expression "effective pharmaceutical quantity", as used herein, is understood as a non-toxic amount, capable of providing a therapeutic effect. The exact amount required will vary from one subject to another, depending on the species, age and general condition of the subject, the severity of the disease being treated, the particular compound used, its mode of administration, and the like. Therefore, it is not possible to specify an exact "effective quantity". However, an appropriate effective amount can be determined by a skilled person through routine experimentation. In the context of compounds and compositions for the prevention of infection, the pharmaceutically effective quantity can refer to the amount necessary to relieve the symptoms of cancer suffered by the subject and improve their quality of life.

Also, in the context of the present invention, "pharmaceutically acceptable excipient" means a therapeutically inactive substance that is said to be used to incorporate the active ingredient, and that is acceptable to the patient from a pharmacological/toxicological point of view and to the pharmaceutical chemist who manufactures it from a physical/chemical point of view, with respect to composition, formulation, stability, patient acceptance and bioavailability.

Another additional object of the invention relates to a food supplement comprising the composition of the invention for use in the treatment of a patient with a solid tumor, including any of the compositions "A-K".

In the context of the present invention, what is understood by food supplement is a food product whose purpose it is to supplement the normal diet, and which consists of concentrated sources of nutrients or other substances, that has a nutritional or physiological effect, in simple or combined form, and is marketed in dosed form, ie capsules, tablets, pills and other similar means, sachets of powders, ampoules of liquid, dropper bottles, and other similar forms of liquids and powders to be taken in small unit quantities, as defined in Directive 2002/46/EC of the European Parliament.

In a particular embodiment of the present invention, the use of the composition as a food supplement comprises orally administering an amount between 100mg to 500mg of the composition, 2 to 6 times a day. Likewise, the use of the composition as an improvement of the state of health in cancer patients is also the object of the invention.

In a particular embodiment of the present invention, the use of the composition as a food supplement comprises orally administering to an individual a dose at least 3 times a day. Also, for the treatment to be effective and to achieve the desired effect, this dose should be administered to the patient at least twice a day and a maximum of 6 times a day. Preferably, the administration of the dose to the patient should be 3, 4 or 5 times a day, ideally 3 times a day.

In another particular embodiment, the amount of the composition is different every time that it is administered to the patient during the day.

In the context of the present invention, a dose is defined as the amount of medicine containing the exact measure of active ingredient so that it is efficient, effective and safe for the patient, and deals with the health problem for which it has been indicated.

Thus, in the context of the present invention, it is established that at least one dose must be administered, preferably at least one daily dose, between 100mg to 80000mg of the composition of the invention, including any of the compositions A-K. In preferred embodiments, a dosage may comprise between 100mg to 50000mg of the composition of the invention, including any of the compositions A-K; in another preferred embodiment, a dosage may comprise between 100mg to 2000mg of the composition of the invention, including any of the compositions A-K; in another preferred embodiment, a dosage may comprise between 100mg to 700mg of the composition of the invention, including any of the compositions A-K. In particular embodiments of the present invention, a dosage may comprise approximately 120mg, 130mg, 140mg, 150mg, 160mg, 170mg, 180mg, 190mg, 200mg 210mg, 220mg, 230mg, 240mg, 250mg, 260mg, 270mg, 280mg, 290mg, 300mg 310mg, 320mg, 330mg, 340mg, 350mg, 360mg, 370mg, 380mg, 390mg, 400mg, 410mg, 420mg, 430mg, 440mg, 450mg, 460mg, 470mg, 480mg, 490mg, 500mg, 510mg, 520mg, 530mg, 540mg, 550mg, 560mg, 570mg, 580mg, 590mg, 600mg, 610mg, 620mg, 630mg, 640mg, 650mg, 660mg, 670mg, 680mg, or 690mg of the composition of the invention, including any of the compositions A-K.

In particular embodiments, where the composition is presented in watertight sachets, this composition can be administered to the patient dissolved or suspended in a liquid, preferably in an aqueous liquid, and more preferably, in beverages such as fruit juices, milk or water. In addition, it can also be mixed with foods such as yogurt, liquid yogurt, soups, purées, creams, or porridge. This food has to be at optimum temperature to be consumed and should never be heated after having added the composition object of the invention.

An additional object of the invention refers to a beverage or food product comprising the composition of oral administration of the invention, or food supplement of the invention.

The technical effect derived from the composition object of the invention is that it acts directly on the host, or by contributing to the reorganization of the microbiota in humans. Thanks to this restorative and modulating action, the inventors have confirmed that, in a surprising way, the fact of intervening in the microbiota of human beings can significantly improve people's state of health, and in particular, from the results in macrophage, one obtains that the activation of the innate immune response is carried out by regulating inflammatory mediators in conditions of inflammation, which would give it the ability to act on cancer cells. Based on the results with zebrafish, one could note that a modulation of angiogenesis is observed in embryonic development that could be assimilated to the tumor. This effect is comparable to that observed with the intestinal microbiota in the early stages of colonization after birth, at the time of formation of the immune system. This proangiogenesis improves the bioavailability in our case of chemotherapeutic and immunotherapeutic treatments, making them more bioaccessible.

In this way, the inventors have been able to verify that the oral administration of the composition object of the invention exerts an effect on the adjustment and reprogramming of the microbiota present in the intestinal tract of people, and that it is not a local effect since the whole organism benefits from this modulation of the microbiota, becoming a systemic effect.

Additionally, the invention comprises the following clauses:

1. A postbiotic composition of oral administration for use in the treatment of solid tumors in a patient, characterized in that said composition comprises lysates of probiotic microorganisms in an amount in percentage by weight with respect to the total weight of the lysates of microorganisms of the composition:

- 6% to 19% of bacterial lysates of the genus *Bacillus;*
- 4% to 8% of bacterial lysates of the genus *Bifidobacterium;*
- 15% to 25% of bacterial lysates of the genus *Lactobacillus;*
- 50% to 60% of yeast lysates of the genus *Saccharomyces;*
- 1.5% to 5% of bacterial lysates of the genus *Streptococcus.*

2. The composition, in accordance with clause 1, comprising lysates of probiotic microorganisms in an amount in percentage by weight of the total weight of the lysates of microorganisms of the composition:

- 13% to 19% of bacterial lysates of the genus *Bacillus;*
- 4% to 8% of bacterial lysates of the genus *Bifidobacterium;*
- 15% to 25% of bacterial lysates of the genus *Lactobacillus;*
- 50% to 60% of yeast lysates of the genus *Saccharomyces;*
- 1.5% to 5% of bacterial lysates of the genus *Streptococcus.*

3. The composition, according to any of the preceding clauses, wherein the composition comprises between 1% and 99.5% by weight of lysates of microorganisms, particularly 5%-99% by weight of lysates of microorganisms, particularly 10%-96% by weight of lysates of microorganisms, 25%-95% by weight of lysates of microorganisms, or particularly 50%±10% by weight of those microorganisms.

4. The composition, according to any of the preceding clauses, wherein the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus, Bacillus paralicheniformes,* and combinations thereof.

5. The composition, according to any of the preceding clausesclauses, wherein the bacterial lysates of the genus *Bacillus* are of the species *Bacillus clausii, Bacillus coagulans, Bacillus licheniformis, Bacillus mesentericus* and *Bacillus subtilis.*

6. The composition, according to any of the preceding clauses, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof.

7. The composition, according to any of the preceding, wherein the bacterial lysates of the genus *Bifidobacterium* are of the species *Bifidobacterium lactis.*

8. The composition, in according to any of the preceding clauses, wherein the bacterial lysates of the genus *Lactobacillus* are of the species that are selected from the group consisting of *Lactobacillus lactis, Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefir* and combinations thereof.

9. The composition, according to any of the precedingclauses, wherein the bacterial lysates of the genus *Lactobacillus* are of the species *Lactobacillus acidophilus, Lactobacillus bulgaricus, Lactobacillus casei, Lactobacillus fermentum, Lactobacillus reuteri* and *Lactobacillus rhamnosus.*

10. The composition, according to any of the preceding clauses, wherein the lysates of the yeast genus *Saccharomyces* are of the species that are selected from the group consisting of *Saccharomyces cerevisiae, Saccharomyces boulardi,* and combinations thereof.

11. The composition, according to any of the preceding clauses, wherein the lysates of the yeast genus *Saccharomyces* are of the species *Saccharomyces cerevisiae.*

12. The composition, according to any of the preceding clauses, wherein the bacterial lysates of the genus *Streptococcus* are of the species that are selected from the group consisting of *Streptococcus thermophilus, Streptococcus salivarius,* and combinations thereof.

13. The composition, according to any of the preceding clauses, wherein the bacterial lysates of the genus *Streptococcus* are of the species *Streptococcus thermophilus.*

14. The composition, according to any of the preceding clauses, wherein the bacterial lysates of the genus *Bacillus* are about 14% to 18% of the total lysates of the composition.

15. The composition, according to any of the preceding clauses, wherein the percentage by weight of *Bacillus licheniformis, Bacillus mesentericus* and *Bacillus subtilis* is approximately the same, and superior to that of *Bacillus clausii* and *Bacillus coagulans,* which is also approximately the same.

16. The composition, according to any of the preceding clauses, wherein bacterial lysates of the genus *Bifidobacterium* are about 5% to 7% of the total lysates of the composition.

17. The composition, according to any of the preceding clauses, wherein the bacterial lysates of the genus *Lactobacillus* are about 20% to 25% of the total lysates of the composition.

18. The composition, according to any of the preceding clauses, wherein the percentage by weight of *Lactobacillus acidophilus, Lactobacillus bulgaricus* and *Lactobacillus casei* is different from each other and different from the percentage by weight of *Lactobacillus fermentum, Lactobacillus reuteri* and *Lactobacillus rhamnosus,* which is approximately identical between these three species.

19. The composition, according to any of the preceding clauses, wherein the percentage by weight of *Lactobacillus casei* is higher than that of the rest of *Lactobacillus* species.

20. The composition, according to any of the preceding clauses, wherein the percentage by weight of *Lactobacillus casei* is higher than that of *Lactobacillus acidophilus,* which in turn is higher than that of *Lactobacillus bulgaricus,* which in turn is higher than that of *Lactobacillus fermentum, Lactobacillus reuteri* and *Lactobacillus rhamnosus,* the percentage by weight of these 3 species being approximately identical.

21. The composition, according to any of the preceding clauses, wherein the lysates of the yeast genus *Saccharomyces* are approximately between 52% to 57% of the total lysates of the composition.

22. The composition, according to any of the preceding clauses, wherein the lysates of the yeast genus *Streptococcus* are approximately between 3% to 4% of the total lysates of the composition.

23. The composition, according to any of the preceding clauses, wherein the composition comprises lysates of probiotic microorganisms in an amount in percentage by weight of the total lysates of the composition according to composition A.

24. The composition, according to any of the preceding clauses, wherein the composition comprises lysates of probiotic microorganisms in an amount in percentage by weight of the total lysates of the composition according to composition B.

25. The composition, according to any of the preceding clauses 1 to 22, wherein the composition comprises lysates of probiotic microorganisms in an amount in percentage by weight of the total lysates of the composition according to composition C.

26. The composition, according to any of the preceding clauses 1 to 22 and 25, wherein the composition comprises lysates of probiotic microorganisms in an amount in percentage by weight of the total lysates of the composition according to composition D.

27. The composition, according to any of the preceding clauses 1 to 22 and 26, wherein the composition comprises lysates of probiotic microorganisms in an amount in percentage by weight of the total lysates of the composition according to composition E.

28. The composition, according to any of the preceding clauses 1 to 22 and 25, wherein the composition comprises lysates of probiotic microorganisms in an amount in percentage by weight of the total lysates of the composition according to composition F.

29. The composition, according to any of the preceding clauses 1 to 22 and 25 and 28, wherein the composition comprises lysates of probiotic microorganisms in an amount in percentage by weight of the total of lysates of the composition according to composition G.

30. The composition, according to any of the preceding clauses 1 to 22 and 25 and 28 to 29, wherein the composition comprises lysates of probiotic microorganisms in an amount in percentage by weight of the total lysates of the composition according to composition H.

31. The composition, according to any of the preceding clauses 1 to 22 and 25 and 28, wherein the composition comprises lysates of probiotic microorganisms in an amount in percentage by weight of the total lysates of the composition according to composition I.

32. The composition, according to any of the preceding clauses 1 to 22 and 25, and 28 and 31, wherein the composition comprises lysates of probiotic microorganisms in an amount in percentage by weight of the total lysates of the composition according to composition J.

33. The composition, according to any of the preceding clauses 1 to 22 and 25, and 28 and 31 to 32, wherein the composition comprises lysates of probiotic microorganisms in an amount in percentage by weight on the total lysates of the composition according to composition K.

34. The composition, according to any of the preceding clauses, which does not include lysates of microorganisms present in the digestive tract, preferably bacterial lysates of the genera *Akkermansia, Enterococcus* and *Escherichia.*

35. The composition, according to any of the preceding clauses, comprising one or more additives selected from the group: carbohydrates (fructose, xylitol, sorbitol, fructooligosaccharides, inulin), antioxidants (resveratrol, beta-carotene), vitamins (vitamin C, D, K, B1, B9, E, B3, B5, A, B3, B6, H, D3, B12 biotin, riboflavin, pyridoxine, pantothenic acid), prebiotics (galacto-oligosaccharides, inulin, oligofructose), amino acids (cysteine, tyrosine, resin, methionine, phenylalanine, glycine, glutamine, alanine, carnitine, glutamine, arginine), lipids (eicosapentaenoic acid, docosahexaenoic acid, arachidonic acid), trace elements (sodium, potassium, magnesium, phosphorus, calcium, copper, zinc, manganese, chromium, iodine, selenium), digestive enzymes (papain, amylase, lactase, bromelain), whey.

36. The composition, according to any of the preceding clauses, comprising one or more of the following additives: resveratrol, astaxanthin, bromelain, papain, fermented rice starch, corn starch and FOS.

37. The composition, according to any of the preceding clauses, that is presented in the form of dry powder.

38. The composition, in accordance with the previous clause, where the dry powder is presented inside watertight sachets or encapsulated in gelatin capsules.

39. The composition, according to any of the preceding clauses, wherein the treatment of solid tumors includes the improvement of the quality of life in the patient.

40. The composition, according to the preceding clause, wherein the improvement of quality of life concerns one or more of the following symptoms: anxiety, tiredness, nausea, vomiting, pain, difficulty sleeping, stress, asthenia, appetite, pain, dyspnea and diarrhea.

41. The composition, according to any of the preceding clauses, wherein the solid tumor is one or more of the following: prostate cancer, breast cancer, cancer of the central nervous system (CNS), pancreatic cancer, esophageal cancer, uterine cancer, lymphoma, pheochromocytoma and sarcoma.

42. The composition, according to the preceding clause, wherein the solid tumor is one or more of the following: prostate, CNS, esophagus, pheochromocytoma and sarcoma.

43. The composition, according to any of the preceding clauses, wherein the patient is a terminal cancer patient.

44. The composition, according to any of the preceding clauses, wherein at least one dose of between 100mg to 700mg is administered, comprising approximately 120mg, 130mg, 140mg, 150mg, 160mg, 170mg, 180mg, 190mg, 200mg 210mg, 220mg, 230mg, 240mg, 250mg, 260mg, 270mg, 280mg, 290mg, 300mg 310mg, 320mg, 330mg, 340mg, 350mg, 360mg, 370mg, 380mg, 390mg, 400mg, 410mg, 420mg, 430mg, 440mg, 450mg, 460mg, 470mg, 480mg, 490mg, 500mg, 510mg, 520mg, 530mg, 540mg, 550mg, 560mg, 570mg, 580mg, 590mg, 600mg, 610mg, 620mg, 630mg, 640mg, 650mg, 660mg, 670mg, 680mg, or 690mg of the composition of the invention, including any of the compositions A-K.

45. The composition, according to any of the preceding clauses, wherein the administration with the postbiotic composition of the invention is daily for at least 2 weeks, preferably daily for at least 3 weeks, more preferably daily for at least 4 weeks, even more preferably daily for at least 5 weeks, even more preferably daily for at least 6 weeks, even more preferably daily for at least 7 weeks, even more preferably daily for at least 8 weeks, or daily for at least 9, or daily for at least 10 weeks, or daily for at least 11 weeks, or daily for at least 12 weeks.

46. The composition, according to any of the preceding clauses, which is administered in an amount between 100mg to 700mg per dose and between 1 to 6 doses a day for at least 2 weeks.

47. The composition, according to any of the preceding clauses, which is administered in an amount between 150 mg to 650 mg per dose and between 1 to 5 doses a day for at least 3 weeks.

48. The composition, according to any of the preceding clauses, which is administered in an amount between 200 mg to 600mg per dose and between 2 to 4 doses a day for at least 4 weeks.

49. The composition, according to any of the preceding clauses, which is administered in an amount between 250 mg to 550mg per dose and between 2 to 4 doses a day for at least 5 weeks.

50. The composition, according to any of the preceding clauses, which is administered in an amount between 300 mg to 500mg per dose and between 2 to 4 doses a day for at least 6 weeks.

51. The composition, according to any of the preceding clauses, which is administered in an amount between 300mg to 500mg per dose and between 2 to 4 doses a day for at least 7 weeks.

52. The composition, according to any of the preceding clauses, which is administered in an amount between 300 mg to 500mg per dose and between 2 to 4 doses a day for at least 8 weeks.

53. The composition, according to any of the preceding clauses, which is administered in an amount of 400mg per dose and 3 doses a day for at least 8 weeks.

54. The composition, according to any of the preceding clauses, wherein the treatment is performed before, simultaneously or after the patient's treatment with chemotherapy or radiotherapy.

55. The composition, according to any of the preceding clauses, wherein the dose of the composition is different each time that it is administered during the day.

56. A procedure for obtaining an orally administered postbiotic composition for use in the treatment of solid tumours in a patient, in accordance with any of clauses 1 to 55 comprising:

- Cultivating the selected microbial species;

- Filtering or centrifuging the microbial species until an adequate biomass of the selected microorganisms is obtained;

- Lysing through cycles of freezing and thawing and sonication of the biomasses obtained.

57. The process, according to the preceding clause comprising drying by atomization or lyophilization of the biomasses of bacterial lysates, obtaining a product in the form of dry powder.

58. A pharmaceutical composition comprising an effective pharmaceutical quantity of the composition, in accordance with any of the clauses 1 to 55, and a pharmaceutically acceptable excipient.

59. A food supplement comprising the composition, in accordance with any of the clauses 1 to 55.

Therefore, the main advantages arising from the composition object of the invention are the following:

- supplement and/or complement the physiological functions of the microbiota of humans and animals;

- the alterations that generate dysbiosis and have an impact on oxidative, inflammatory and antitumor processes are restored;

- the fact that the administration of the product is oral, presents the composition and its surprising effect as a very advantageous alternative, compared to cancer treatments currently used in medicine;

- The present composition includes lysates of microorganisms, that is, it does not present living organisms, thus the composition is presented as a safe alternative because it would avoid the possible dangers of colonizing organisms, and also does not present the toxicity that a conventional drug can present.

Each of the terms "comprising", "consisting essentially of" and "consisting of" may be replaced by either of the other two terms. The term "a" may refer to one or a plurality of the elements they modify (for example, "a reactant" may mean one or more reactants), unless it is contextually clear that one or more than one of the elements is described. The term "approximately," as used herein, refers to a value within 10% of the underlying parameter (i.e., plus or minus 10%; for example, a weight of "approximately 100 grams" may include a weight between 90 grams and 110 grams). The use of the term "approximately" at the beginning of a list of values modifies each of the values (for example, "approximately 1, 2, and 3" refers to "approximately 1, approximately 2, and approximately 3"). When describing a list of values, the list includes all intermediate values and all fractional values thereof (for example, the list of values "80%, 85% or 90%" includes the intermediate value 86% and the fractional value 86.4%). When a listing of values is followed by the term "or more", the term "or more" is applied to each of the values listed (for example, the listing of "80%, 90%, 95% or more" refers to "80% or more, 90% or more, or 95% or more"). When describing a list of values, the listing includes all ranges between two of the listed values (for example, the listing of "80%, 90%, or 95%" includes ranges of "80% to 90%," "80% to 95%," and "90% to 95%"). Below are some examples of the application of the technology.

## PREFERRED EMBODIMENT OF THE INVENTION

So as to contribute to a better understanding of the invention, and in accordance with a practical realization thereof, a preferred embodiment of the present invention will be detailed as an integral part of this description.

## EXAMPLE 1 - IMMUNOMODULATORY ACTIVITY OF A LYSATE OF MICROORGANISMS IN THP-1 CELLS

### • Objective:

The aim of this study is to investigate the immunomodulatory potential, in vitro, of a microorganism lysate by regulating cytokine secretion in THP-1 cells.

### • Materials and Methods:

Composition of the postbiotic invention:

A postbiotic composed of lysates from 14 strains/species *of Lactobacillus sp., Bifidobacterium sp., Bacillus sp, Saccharomyces sp.* and *Streptococcus sp. was* used. The composition of the extracts appears in Table 2 in an amount in percentage by weight over the total weight of the lysates of the composition.

Table 2: Components of the composition of the invention

| Species/Strains | % (w/w) |
|---|---|
| *Bacillus clausii* | 0,3 |
| *Bacillus coagulans* | 0,3 |
| *Bacillus licheniformis* | 5 |
| *Bacillus mesentericus* | 5 |
| *Bacillus subtilis* | 5 |
| *Bifidobacterium lactis* | 5 |
| *Lactobacillus acidophilus* | 3,7 |
| *Lactobacillus bulgaricus* | 3,6 |
| *Lactobacillus casei* | 5 |
| *Lactobacillus fermentum* | 3 |
| *Lactobacillus reuteri* | 3 |
| *Lactobacillus rhamnosus* | 3 |
| *Saccharomyces cereviseae* | 55 |
| *Streptococcus thermophilus* | 3 |

Macrophage culture:

Macrophages were obtained from the THP-1 cell line (human monocyte cell line, ATCC® TIB-202). These cells were stored in Gaiker's culture bank and checked for the absence of contaminating mycoplasmas immediately after thawing. The cells were grown in suspension at 37°C in a humid atmosphere of 5% $CO_2$. They were kept in culture medium RPMI + 10 % FBSi + 50 $\mu$M $\beta$-mercaptoethanol, for a minimum of two weeks before any experiment. The cells were subcultured before reaching 80% confluence (cell concentration/volume).

A cell suspension of $1*10^5$ cells/mL was prepared 24 hours before the immunomodulatory activity test. $1*10^4$ cells/well were dispensed in 96-well plates in the presence of 0.31 $\mu$g/mL PMA to differentiate monocytes from macrophages. The plates were incubated at 37°C and 5% $CO_2$ for 24 hours.

Immunomodulatory activity test:

For the immunomodulation test, THP-1 PMA-differentiated cells with different concentrations of the postbiotic composition of Table 2 were incubated for 24 hours in the presence of an inflammatory stimulus, lipopolysaccharide, LPS (20ug/ml). Non-inflamed cells (cells exposed to unstimulated culture medium) were used as a negative control. The supernatant was collected after 24 hours. Levels of cytokines IL-8, TNF-$\alpha$, IL-6 and IL-18 were quantified using an ELISA detection kit in accordance with the manufacturer's instructions (Human IL-8 Elisa Kit. Invitrogen: KHC0082; Human IL-6 Elisa Kit. Invitrogen: KHC0062; Human IL-18 Elisa Kit. MBL: 7620; Human TNF-alpha Elisa Kit. Invitrogen; 10008932).

Cytotoxicity study:

To study the cytotoxicity of the products, a viability test was performed by MTT. MTT, (a yellow tetrazolium), is reduced to purple formazan in living cells, evaluating cellular metabolic activity and, therefore, cell viability. The cells grown in 96-well plates were treated with 8 concentrations of the products understudy for 24 hours (37°C, 5% CO2). In parallel, 8 concentrations of a positive control (sodium dodecyl sulfate (SDS)) were also tested to validate the study. After 24 hours of incubation with the postbiotic or positive control, the cells were washed with PBS and stained with a solution of MTT incubating at 37°C for 2 hours. At the end of this incubation period, the staining medium was removed and 100 $\mu$l of DMSO/well was added to solubilize the colored precipitate. The absorbance was read at 540 nm in the spectrophotometer plate reader. The percentage of cell viability was calculated in relation to the negative control, a condition in which the cells were not exposed to any product.

$$\% \text{ cell viability} = AbT/AbC \times 100$$

AbT = absorbance at 540 nm after 72 hours of treatment
AbC = absorbance at 540 nm after 72 hours without treatment (negative control)

• **Results**

Mycoplasma test

The results obtained in the mycoplasma test are shown in the following table:

Table 3. Ratio of mycoalert obtained in each cell line

| Mycoalert ratio obtained in each cell line | | | |
|---|---|---|---|
| Cell | Reading A | Reading B | Ratio (reading B/reading A) |
| THP-1 | 3503 | 1265 | 0.361 |

The ratios obtained in the MycoAlert® mycoplasma detection kit (Lonza LT07-318) were less than 0.9, indicating the absence of mycoplasma contamination.

Cytotoxicity test

The cytotoxic effect of the compound was tested in vitro, by MTT assay, in the THP-1 cell line. For this, eight concentrations of the compound under study were analyzed: 500 $\mu$g/mL, 250 $\mu$g/mL, 125 $\mu$g/mL, 62.5 $\mu$g/mL, 31.25 $\mu$g/mL, 15.63 $\mu$g/mL, 7.81 $\mu$g/mL and 3.91 $\mu$g/mL. In parallel, different concentrations of sodium dodecyl sulfate (SDS) were used as a positive control (not shown).

Figure 1 shows that the concentrations 15.63 $\mu$g/mL, 7.81 $\mu$g/mL and 3.91 $\mu$g/mL did not alter the viability of THP-1 cells and were therefore selected for the immunomodulation test.

Immunomodulatory activity

THP-1 cells differentiated to macrophages were exposed simultaneously to an inflammatory stimulus with LPS and to different concentrations of the postbiotic according to the cytotoxicity results, during 24 h. After exposure time, supernatants were collected and analyzed. The immunomodulatory effect was analyzed by quantifying the secreted levels of IL-6, IL-8, IL-18 and TNF-$\alpha$. The results obtained are represented in Figure 2. All concentrations tested induce an increase in the level of cytokines IL-8 and IL-6. The results show that in macrophages treated with LPS and postbiotic, they are secreted at a higher value than that observed in macrophages only stimulated with LPS. The levels of IL-8 and IL-6 produced after 24 h remained high for all concentrations tested.

The secretion of TNF-$\alpha$ and IL-18 was also analyzed, the results of which are summarized in Figure 2. In this case the levels were similar for all the concentrations tested, however in all cases a lower secretion was observed than that observed only with LPS.

### • Conclusion

The study shows that the postbiotic composition of the invention under the conditions described, presents immunomodulatory activity in all concentrations tested (15.63 $\mu$g / mL, 7.81 $\mu$g / mL and 3.91 $\mu$g / mL). LPS stimulation increases the production of IL-6, IL-8, TNF-$\alpha$ and IL-18 in THP-1 cells. When inflamed cells are also treated with this composition, the secreted levels of IL-18 and TNF-$\alpha$, compared to cells treated only with LPS, decrease. On the other hand, the response of the cells when also treated with the composition increased the response by increasing the secretion of IL-8 and IL-6 with respect to only LPS.

The results of this study could indicate that the composition of the invention provides an immunomodulatory effect by activating the innate immune response of THP-1 macrophages by regulating pro-inflammatory mediators.

### EXAMPLE 2: EVALUATION OF THE ANGIOGENIC EFFECT OF THE POSTBIOTIC IN ZEBRAFISH.

The need for the formation of new blood vessels is important during embryogenesis and in pathological situations such as tumor growth. Angiogenesis is a cellular process by which already established vessels branch out and invade tissues in response to normal or pathological stimuli. Many processes and regulatory factors that control embryonic angiogenesis are shared by tumor-induced angiogenesis. Zebrafish have a complex circulatory system similar to that of mammals, and its use has been validated to detect the anti-angiogenic or pro-angiogenic activity of a compound. The existence of transgenic zebrafish that express a green fluorescent protein (GFP) under specific promoters of the vascular system, allows us to directly observe the development of blood vessels and any alterations under a fluorescence microscope.

The objective of this study is to evaluate the effect of molecules of microbial origin on angiogenesis in zebrafish embryos. For this, a dose range search (DRF) study was first conducted for toxicity evaluation, and then the active ingredients were tested to study their effect on intersegmental vessel formation (ISV).

### • Study design

The trial used a transgenic zebrafish (*Danio rerio*), which expresses the GFP protein in blood vessels under the promoter Flk1. The strain of origin was wild type AB. The study used 140 embryos from BIOBIDE. Adult zebrafish were housed and kept following standard procedures (ZFIN). The compound to be analyzed was the composition of the postbiotic invention of table 2 (IGEN-1806), preserved at 4°C and resuspended in DMSO. The positive control was a selective vascular endothelial growth factor receptor (VEGFR) inhibitor, KRN633, from MedChem Express in DMSO.

1. Dose range search (DRF):

Five concentrations, 0.1, 1, 10, 100 and 1000 mg/l, were used with 10 embryos per condition (5 embryos per well) incubating for 2 days at 28.5 °C. The embryos were treated at 24 $\pm$ 1 hpf. The lethality, morphological alterations and signs of angiogenesis alteration/neovascularization were evaluated at 48 and 72 hpf. The control were untreated embryos in E3 + 0.5% DMSO medium. The synchronized embryos were kept at 28.5 °C in an incubator until they reached 24 $\pm$ 1 hpf. The lysate dissolved in DMSO at a concentration of 200 ug/l, storing it at 2-8 °C, and from this, dilutions were prepared (0.1, 1, 10, 100 and 1000 mg / l).

At approximately 23 hpf, chorions from healthy, fertilized embryos were placed in 24-well plates (5 embryos per well) in 995 $\mu$l of E3 medium. Next, 5 $\mu$l of postbiotic or DMSO was added in the case of the control and the plates were incubated at 28.5 °C. Embryos were analyzed at 48 and 72 hpf under the stereoscope. The incidence of lethality and the presence of clear morphological alterations (affecting craniofacial structures, trunk and tail), edema and other anomalies were recorded. They were also analyzed under fluorescence stereoscope in the same stages. Signs of neovascularization (ectopic growth) were visually checked in ISVs (from 48 hpf, embryos were anesthetized with tricaine (0.03 mg/ml) for proper analysis).

The experiment was considered valid when the number of unaltered embryos (n) (normal morphology and properly developed ISVs) in the control group was equal to 8 (n=80%).

The active ingredients are considered non-toxic at the doses analyzed, when the number of affected embryos (dead or with altered morphology) represents less than 20% of the total embryos treated.

2. Angiogenesis inhibition assay:

Six selected concentrations based on the previous DRF analysis (700, 1000, 1200, 1500, 1700 and 2000 mg/l) were used with 10 embryos per condition (5 embryos per well) incubating for 2 days at 28.5 °C. The positive control were embryos treated with 50 nM KRN633 in E3 medium (0.5% DMSO). The embryos were treated at 24 hpf. The incubation time was 48 hours at 28.5 °C. The control group consisted of untreated embryos in E3 + 1% DMSO medium. ISV formation was evaluated at 48 and 72 hpf. Embryo production is described in the DRF procedure.

The embryos were dechorionated, dispensed and treated as described in the DRF procedure. After 24 hours of incubation at 28.5 °C, 48 hpf embryos were removed from the exposure medium, anesthetized with 0.03% tricine, and once asleep, examined for possible defects in ISV development. A fluorescent image of each embryo of the different experimental groups was taken with a fluorescent stereomicroscope. Subsequently, image analysis was performed, and the total number of ISVs presented in the trunk of the embryos (from where the bulkiest part of the yolk ends to the tip of the tail) and the number of ISVs that were complete were quantified, which means that they reach the DLAV (Dorsal Longitudinal Anastomotic Vessel) without interruptions. Since defects in ISV germination were not present or were not clearly detected at 48 hpf, embryos were washed with E3 medium to remove tricaine, placed back into the exposure medium and incubated for a further 24 hours at 28.5 °C to check vasculature development at 72 hpf. At this stage, circulation through ISVs was examined, as well as the integrity of ISVs and DLVs. The number of embryos presenting a characteristic vascular phenotype for each experimental group was quantified.

The percentage of embryos in the control group that showed properly developed ISVs was greater than 80%, the experiment was considered valid. In addition, the induction of statistically significant ISV defects in positive control embryos was considered for validation.

To consider that the postbiotic composition of the invention has a role in angiogenesis, the value of any of the parameters quantified in the analysis of the formation of intersegmental vessels at any time analyzed during the study must be significantly different ($p < 0.05$) when compared with control embryos.

• Results of the dose range search trial

The results obtained after the treatment of zebrafish embryos to evaluate their effect on angiogenesis are shown in Table 4. The numbers presented in each table indicate the number of embryos showing no apparent alteration, affected embryos and dead embryos respectively.

Table 4. Summary of unaffected, affected or dead embryos (i.e. (8/2/0) after treatment with 5 doses of the composition of the invention

| Day of the analysis | | 48 hpf | | | | | |
|---|---|---|---|---|---|---|---|
| Concentrations (mg/l) | | Control | 0.1 | 1 | 10 | 100 | 1000 |
| Identification Test | | | | | | | |
| IGEN-1806 | PE-3818 | 8/2/0 | 10/0/0 | 10/0/0 | 10/0/0 | 9/1/0 | 10/0/0 |
| | | | | | | | |
| Day of the analysis | | 72 hpf | | | | | |
| Concentrations (mg/l) | | Control | 0.1 | 1 | 10 | 100 | 1000 |
| Identification Test | | | | | | | |
| IGEN-1806 | PE-3818 | 8/2/0 | 10/0/0 | 10/0/0 | 10/0/0 | 8/2/0 | 10/0/0 |

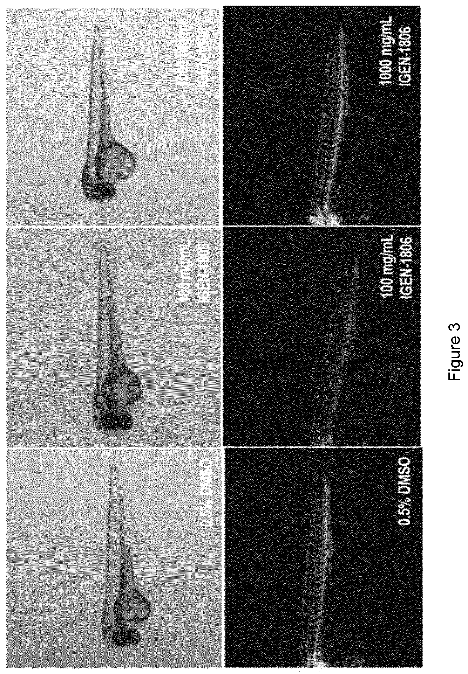

Embryonic morphology was not affected in most of the conditions analyzed. However, one of the fish treated at 100 mg/l showed yolk opacity and yolk edema at 48 hpf, and the same fish also showed abnormal length and cardiac edema at 72 hpf, while another fish of the same dose showed only heart edema at 72 hpf. In addition to this morphological analysis, embryos were examined under the fluorescence stereoscope, and both ISVs and DLAVs were adequately formed at all concentrations and stages analyzed (see Figure 3).

Based on these results, the concentrations selected for the next phase of the study were 700,1000,1200,1500,1700 and 2000 mg/l.

• Results of the angiogenesis inhibition assay

Analysis at 48 hpf.

An analysis of possible angiogenesis defects caused by the postbiotic was performed and photographs of each were taken. Fish were taken in all experimental groups for ISV quantification. The raw data of the total and complete number of vessels, the mean values, the standard deviation (SD), the standard error of the mean (SEM), the results of the statistical analysis and the representative images are presented in the following tables, and in graphs and figures enclosed in this document.

Table 5. Number of total ISVs.

| 48 hpf Fish | Control (1%DMSO) | | KRN633 50 Nm | |
|---|---|---|---|---|
| | Total ISVs | Complete | Total ISVs | Complete |
| 1 | 21 | 18 | 15 | 0 |
| 2 | 20 | 14 | 15 | 0 |
| 3 | 21 | 17 | 15 | 0 |
| 4 | 21 | 21 | 15 | 0 |
| 5 | 21 | 16 | 16 | 16 |
| 6 | 21 | 20 | 15 | 0 |
| 7 | 22 | 16 | 15 | 0 |
| 8 | 22 | 16 | 16 | 0 |
| 9 | 22 | 17 | 15 | 0 |
| 10 | 21 | 12 | 16 | 0 |

Table 6. Number of complete ISVs quantified in the experimental groups indicated at 48 hpf.

| 48 hpf Fish | 700 mg/l | | 1000 mg/l | | 1200 mg/l | | 1500 mg/l | | 1700 mg/l | | 2000 mg/l | |
|---|---|---|---|---|---|---|---|---|---|---|---|---|
| | Total ISVs | Complete | Total ISVs | Complete | Total ISVs | Complete | Total ISVs | Complete | Total ISVs | Complete | Total ISVs | Complete |
| 1 | 22 | 19 | 22 | 16 | 21 | 21 | 23 | 22 | 23 | 17 | 23 | 21 |
| 2 | 22 | 20 | 22 | 20 | 20 | 20 | 21 | 17 | 20 | 20 | 23 | 19 |
| 3 | 22 | 15 | 22 | 19 | 22 | 16 | 23 | 20 | 22 | 22 | 22 | 22 |
| 4 | 22 | 22 | 22 | 17 | 23 | 18 | 23 | 20 | 22 | 22 | 21 | 18 |
| 5 | 21 | 19 | 21 | 15 | 22 | 18 | 22 | 21 | 22 | 21 | 21 | 19 |
| 6 | 21 | 13 | 20 | 18 | 21 | 18 | 22 | 19 | 22 | 21 | 21 | 20 |
| 7 | 22 | 15 | 20 | 16 | 22 | 21 | 22 | 17 | 23 | 23 | 21 | 21 |
| 8 | 21 | 20 | 22 | 17 | 21 | 17 | 22 | 19 | 22 | 22 | 21 | 20 |
| 9 | 22 | 16 | 22 | 18 | 22 | 22 | 22 | 17 | 22 | 20 | 21 | 20 |
| 10 | 22 | 19 | embrión muerto | | 22 | 19 | 22 | 17 | embrión muerto | | 22 | 20 |

The statistical analysis is shown in the following table:

Table 7. Mean, SD, SEM and statistical analysis results of ISV results at 48 hpf. ns: not significant; *: p<0.05, **: p>0.01; ***: p<0.001

| 48 hpf | | Control | 50 nM | Postbiotico | | | | | |
|---|---|---|---|---|---|---|---|---|---|
| | | (1%DMSO) | KRN633 | 700 mg/l | 1000 mg/l | 1200 mg/l | 1500 mg/l | 1700 mg/l | 2000 mg/l |
| TOTAL | Mean | 21,2 | 15,3 | 21,7 | 21,44 | 21,6 | 22,2 | 22 | 21,6 |
| | SD | 0,63 | 0,48 | 0,48 | 0,88 | 0,84 | 0,63 | 0,87 | 0,84 |
| | SEM | 0,2 | 0,15 | 0,15 | 0,29 | 0,27 | 0,2 | 0,29 | 0,27 |
| | ANOVA | | *** | ns | ns | ns | * | ns | ns |
| COMPLETE | Mean | 16,7 | 1,6 | 17,8 | 17,33 | 19 | 18,9 | 20,89 | 20 |
| | SD | 2,63 | 5,06 | 2,86 | 1,58 | 1,94 | 1,85 | 1,76 | 1,15 |
| | SEM | 0,83 | 1,6 | 0,9 | 0,53 | 0,61 | 0,59 | 0,59 | 0,37 |
| | ANOVA | | *** | ns | ns | ns | ns | ** | * |

These results show that the composition of the invention produced an alteration in the number of complete ISVs at 48 hpf at 1700 and 2000 mg/l.

In addition, a significant increase (p<0.05) in the total number of ISVs was also observed at 1500 mg/ml.

Analysis at 72 hpf.

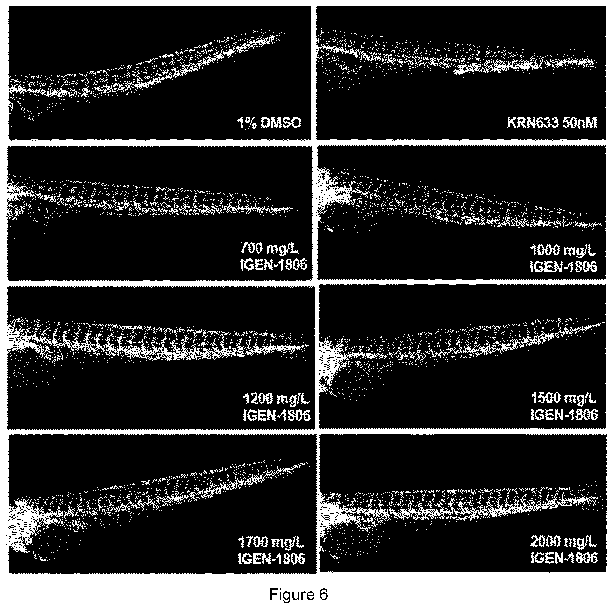

Since some defects in the development of ISVs were observed at 48 hpf, embryos were also tested at 72 hpf for ISV and DLAV integrity. Few embryos did not survive this stage, more specifically those exposed to the highest concentration (2000 mg/l). The number of ISVs and complete ISVs for each experimental group was quantified and the results obtained are shown in the following tables and Figure 6.

Table 8. Number of total ISVs.

| 72 hpf Fish | Control (1%DMSO) | | KRN633 50 Nm | |
|---|---|---|---|---|
| | Total ISVs | Complete | Total ISVs | Complete |
| 1 | 21 | 20 | 15 | 8 |
| 2 | 21 | 20 | 16 | 6 |
| 3 | 22 | 21 | 14 | 9 |
| 4 | 21 | 20 | 13 | 7 |
| 5 | 22 | 19 | 12 | 6 |
| 6 | 20 | 19 | 13 | 5 |
| 7 | 21 | 20 | 14 | 7 |
| 8 | 21 | 20 | 14 | 10 |
| 9 | 21 | 21 | 15 | 8 |
| 10 | 21 | 18 | 14 | 6 |

Table 9. Number of complete ISVs quantified in the indicated experimental groups at 72 hpf

| 48 hpf Fish | 700 mg/l | | 1000 mg/l | | 1200 mg/l | | 1500 mg/l | | 1700 mg/l | | 2000 mg/l | |
|---|---|---|---|---|---|---|---|---|---|---|---|---|
| | Total ISVs | Complete | Total ISVs | Complete | Total ISVs | Complete | Total ISVs | Complete | Total ISVs | Complete | Total ISVs | Complete |
| 1 | 21 | 18 | 21 | 19 | 21 | 19 | 21 | 17 | 21 | 21 | 22 | 20 |
| 2 | 21 | 21 | 20 | 18 | 22 | 19 | 22 | 18 | 22 | 21 | 22 | 15 |
| 3 | 20 | 18 | 22 | 21 | embrión muerto | | 22 | 21 | 21 | 20 | 20 | 18 |
| 4 | 21 | 15 | 21 | 20 | 19 | 16 | 22 | 21 | 22 | 21 | 22 | 14 |
| 5 | 21 | 21 | 22 | 22 | 22 | 20 | 22 | 16 | 20 | 19 | 21 | 20 |
| 6 | 21 | 19 | 20 | 19 | embrión muerto | | 21 | 17 | 21 | 16 | 21 | 20 |
| 7 | 19 | 19 | 20 | 20 | 22 | 19 | 21 | 20 | 21 | 10 | embrión muerto | |
| 8 | 21 | 20 | 20 | 18 | 20 | 18 | 21 | 10 | 21 | 18 | embrión muerto | |
| 9 | 21 | 20 | 22 | 22 | 21 | 17 | 20 | 17 | 22 | 14 | embrión muerto | |
| 10 | embrión muerto | | embrión muerto | | 21 | 20 | 21 | 13 | embrión muerto | | embrión muerto | |

Table 10. Media, SD, SEM and results of satisfied analysis of ISV results for treated embryos and controls at 72 hpf.
ns; not significant :* p<0.05, **: p<0.01;; p<0.001

| 72 hpf | | Control | 50 nM | Postbiotico | | | | | |
|---|---|---|---|---|---|---|---|---|---|
| | | (1%DMSO) | KRN633 | 700 mg/l | 1000 mg/l | 1200 mg/l | 1500 mg/l | 1700 mg/l | 2000 mg/l |
| TOTAL | Mean | 21,1 | 14 | 20,67 | 20,89 | 21 | 21,3 | 21,22 | 21,33 |
| | SD | 0,57 | 1,15 | 0,71 | 0,93 | 1,07 | 0,67 | 0,67 | 0,82 |
| | SEM | 0,18 | 0,37 | 0,24 | 0,31 | 0,38 | 0,21 | 0,22 | 0,33 |
| | ANOVA | | *** | ns | ns | ns | ns | ns | ns |
| COMPLETE | Mean | 19,8 | 7,2 | 19 | 19,89 | 18,5 | 17 | 17,78 | 17,83 |
| | SD | 0,92 | 1,55 | 1,87 | 1,54 | 1,41 | 3,46 | 3,8 | 2,71 |
| | SEM | 0,29 | 0,49 | 0,62 | 0,51 | 0, 5 | 1,1 | 1,27 | 1,11 |
| | ANOVA | | *** | ns | ns | ns | ns | ns | ns |

These results indicated that the composition of the invention did not alter the development of ISVs at any of the concentrations testes at 72 hpf. neither in the numer of ISVs nor in the complete ISVs.

• **Conclusion**

The objective of this study was to determine the anti- or pro-angiogenic potential of the composition of the invention in zebrafish embryos. After analyzing the toxicity and vascular effects affecting vessel formation at 24 and 48 hours after treatment, an analysis of the number and integrity of ISVs was performed. The results indicate that the postbiotic mix did not induce a clear antiangiogenic effect in zebrafish embryos at 72 hpf under the conditions used. However, at 48 hpf, a moderate increase in the number of complete ISVs was observed compared to control, especially at higher doses. This could indicate a slight induction of angiogenesis formation only in the early stages of development.
In relation to solid tumors, growth and metastasis is achieved by excessive angiogenesis, that results in the creation of blood vessels with an abnormal structure that generate an irregular perfusion which alters the distribution of nutrients and oxygen, as well as the administration of chemotherapy drugs. This reduces the effect of treatments to reach the tumor tissue. For this reason it has recently been proposed that using pro-angiogenic agents can induce the formation of new healthy blood vessels and heal abnormal blood vessels throughout the tumor microenvironment. A recent study has revealed that stimulating angiogenesis can sensitize tumors to chemotherapy and that the combination of verapamil and cilengitide increased vessel density, dilation, patency, and perfusion within tumors. This in turn, increased the degree of oxygenation of the tumor and allowed for more efficient and more effective delivery of the chemotherapeutic drug gemcitabine. Therefore, proangiogenesis may also serve as a potential therapy in the treatment of cancer.
The composition of the invention presents the ability to induce the formation of angiogenesis only in the early stages of development, which allows us to conclude that it could slow down the formation process, by improving the accessibility of drugs and nutrients, and limiting hypoxia, thus preventing the tumor process.

**EXAMPLE 3:**

**ADMINISTRATION OF THE POSTBIOTIC COMPOSITION OF THE INVENTION AS A MEANS TO REGULATE THE MICROBIOTA IN TERMINAL CANCER PATIENTS.**

• **Objective:**

What is analyzed is whether the composition of the invention can re-balance the composition and physiological function of the microbiota of terminal cancer patients, and if this modification is accompanied by clinical and quality of life improvement, and of the cachexia that usually accompanies these patients.

• **Study Design:**

Prospective observational study of a series of 34 terminal-stage patients with different types of cancer (Table 11), with theoretical life expectancy by stage and treatment of less than 6 months. Demographic data, form of presentation, diagnosis and treatment received are collected.

The inclusion criterion was patients of both sexes, over 18 years of age, cancer patients of any location, in an advanced stage of metastasis, and theoretical life expectancy of less than six months, with any type of active treatment. Patients in non-advanced stages of the disease, whose quality of life could not be assessed or who were receiving another type of food supplement or special nutrition, and children under 18 years of age, were excluded. All patients were administered a dose of the composition of the invention of 400 mg, three times a day, that is, 1200 mg daily, (3 daily doses) for at least 8 weeks, and quality of life was measured by validated EORTC QLQ-C30 questionnaire, along with side effects of chemotherapy, anorexia, weight gain/loss and asthenia. Before starting the treatment, and then weekly, patients had to fill out the questionnaire and it was handed-in at subsequent visits. The composition of the invention comprised three sachets of 10g, each sachet containing 400 mg of the composition of lysed microorganisms of the invention. The rest were excipients and additives such as Whey 5.44g, Calcium 200 mg, Vitamin C 26.67 mg, Bromelain 24 mg, Papain 32.5 mg, Vitamin B1 0.367 mg, Copper 0.33 mg, Vitamin B9 66.67ug, Fructooligosaccharides 280 mg, Inulin 238 mg, L-Carnitine 173 mg, L-Glutamine 135 mg, L-Arginine 102 mg, Resveratrol 2.5 mg, Vitamin E 4 mg, Vitamin B3 5.33 mg, Vitamin B5 2 mg, Vitamin A 266.67 ug, Vitamin B2 0.467 mg, Vitamin B6 0.467 mg, Vitamin H 16.67 ug, Vitamin D3 1.67 ug, Vitamin B12 0.83ug, Potassium 260 mg, Magnesium 125 mg, Sodium 86.67 mg, Zinc 3.33 mg, Manganese 0.67mg, Chromium 13.33 ug, Iodine 50 ug, Selenium 18.33ug.

The contents of one sachet (10 g) were poured into a glass, water (approximately 150 ml) was added and stirred until complete dissolution. Each patient ingested three doses a day shortly before meals, approximately 20 minutes before each main meal (breakfast, lunch and dinner).

Table 11: Summary table of patients included in the study

|  | Sample | Averag e Age | % Males | Postbiotic only | %Postbioti c only |
|---|---|---|---|---|---|
| **OTHER TUMORS** | 15 | 60 | 53% | 4 | 27% |
| **PROSTATE** | 7 | 74 | 100% | 1 | 14% |
| **BREAST** | 2 | 44 | 0% | 0 | 0% |
| **CNS** | 2 | 30 | 67% | 1 | 50% |
| **PANCREAS** | 1 | 61 | 40% | 0 | 0% |
| **ESOPHAGUS** | 1 | 58 | 100% | 1 | 100% |
| **UTERUS** | 2 | 59 | 0% | 0 | 0% |
| **LYMPHOMA** | 2 | 36 | 100% | 0 | 0% |
| **PHEOCHROMOCYTOM A** | 1 | 48 | 100% | 1 | 100% |
| **SARCOMA** | 1 | 50 | 0% | 1 | 100% |
| **TOTAL** | 34 | 51 |  | 9 | 26% |

• **Results:**

80% presented stabilization or weight gain with respect to baseline, even in those patients who had cachexia when they started the study.

In 56% of patients receiving concomitant chemotherapy, the digestive side effects of chemotherapy (nausea, vomiting, diarrhea) were reduced or disappeared. Asthenia was one of the parameters that improved the most throughout the study. Although it was not one of the study parameters, the life expectancy of 71% of the treated patients was higher than expected for the stage of their illness. A partial or complete radiological response to the lesions occurred in 23% of patients.

For the statistical assessment, we chose to use the McNemar statistical test for paired data, applying continuity correction. To that end, it was considered that the patient presents improvement when the standard error of the mean (SEM) was higher than Cronbach's Alpha. Table 12 shows the summary of significance data for each of the scales. One can observe that all the scales are statistically significant.

Table 12. Summary of significance data for each of the scales.

| | PF | RF | EF | CF | SF | QL | FA | |
|---|---|---|---|---|---|---|---|---|
| Z McNemar cont correc | 3,491 | 3,671 | 3,198 | 1,455 | 2 | 2.694 | 4,085 | |
| Q< | 0,005 | 0,005 | 0,01 | 0,15 | 0,04 | 0,01 | 0,005 | |
| | | | | | | | | |
| | NV | PA | DY | SL | AP | CO | DI | FI |
| Z McNemar cont correc | 2,25 | 2,907 | 1,443 | 2,919 | 3,055 | 1,336 | 0 | 0 |
| Q< | 0,03 | 0,01 | 0,15 | 0,01 | 0,005 | 0,18 | | |

(PF) Physical, (RF) Role, (EF) Emotional, (CF) Cognitive, (SF) Social, (QL) Global, (FA) Fatigue, (NV) Nausea and Vomiting, (PA) Pain, (DY) Dyspnea, (SL) Sleep disturbance, (AP) Appetite, (CO) Constipation, Diarrhea (DI), Financial impact (FI).

Treatment was associated with statistically significant improvement in all the scales of the questionnaire except for the cognitive scale (CO), dyspnea (DY) and the constipation/diarrhea (FC) binomial. The scales that obtained the greatest power of significance were the physical (PF) and role (RF) scales, and the symptoms of fatigue (AF) and loss of appetite (PA).

During the study, 97% of patients showed a substantial improvement (greater than Cronbach's alpha) in their quality of life with respect to the baseline situation prior to starting, in some of the functional scales, or in the symptoms of the questionnaire. The average improvement was 34 points, an improvement considered very important according to clinical trials of validation of the EORTC-QLQ-C30 questionnaire (https://qol.eortc.org/questionnaire/eortc-qlq-c30/). To consider that a patient had improvement in each of the functional scales or symptoms, the SEM and Cronbach's Alpha were estimated, and a value that exceeded said Alpha for this scale was accepted as improvement.

Additionally, 62% of patients presented an improvement in the total assessment of the questionnaire of 33 points, which is also considered an important improvement.

The symptoms or functional scales that improved in the highest percentage of patients were fatigue (76%), sleep disturbances (56%) and appetite (53%), which was associated with a clear improvement in the physical (56%) and emotional (56%) scales. On the other hand, dyspnea improved in 26% of patients, but in those concerned, the rate of improvement was the highest of the entire study with an average of 56 points (min 33, max. 100). The other two symptoms that improved with rates of more than 50 points were diarrhea and constipation. Nausea and vomiting improved in more than a third of the patients (38%), with significant improvements of almost 30 points on average.

All these values were surpassed at week 12, in those patients who continued with the treatment and submitted questionnaires voluntarily since the study was completed at 8 weeks.

The magnitude of change in the different scales included in the questionnaire has been estimated using the effect size (ES), the standardized mean response (SMR) and the minimally important difference (MID), or minimal clinically important difference (MCID).

The effect size (ES) was calculated by dividing the differences between the mean responses observed before and after the pharmacological intervention (8 weeks of intake) by the standard deviation observed at baseline. The standardized mean response (SMR) was calculated by dividing the mean change between the initial and final visits by the standard deviation of the change. The reference values for SMR (the well-known Cohen thresholds) with regard to its use as a measure of effect are:

1. Non-important effect: SMR<0.20

2. Small effect: SMR >0.20<0.50

3. Moderate effect: SMR >0.5<0.8

4. Large effect: SMR >0.80

As can be seen in the table, the magnitude of the effect is moderate or large for all the scales, except cognitive and social among functional scales, and diarrhea and dyspnea among symptom scales, something that is related to the level of significance of said scales, as we saw above. Although the data at 12 weeks has not been evaluated for this study, it should be noted that the effect becomes large on most scales, which allows us to predict a greater impact of effect if the treatment is longer. As can be seen from the results, the administration of the composition for a long period (3 times a day, for 12 weeks at least) hardly presents any side effects, so the invention is extremely safe.

Table 13. Magnitude of effect.

|  | 4wk EFFECT | | 8wk EFFECT | | 12wk EFFECT | |
|---|---|---|---|---|---|---|
|  | ES | SMR | ES | SMR | ES | SMR |
| **Physical (PF)** | 0,38 | 0,39 | 0,66 | 0,7 | 0,89 | 1,12 |
| **Role (RF)** | 0,39 | 0,45 | 0,61 | 0,68 | 0,81 | 0,77 |
| **Emotional (EF)** | 0,37 | 0,37 | 0,6 | 0,73 | 0,8 | 1,02 |
| **Cognitive (CF)** | 0,04 | 0,05 | 0,16 | 0,17 | 0,28 | 0,25 |
| **Social (SF)** | 0,25 | 0,27 | 0,28 | 0,29 | 0,32 | 0,34 |
| **Global (QL)** | 0,44 | 0,46 | 0,6 | 0,65 | 0,73 | 0,8 |
| **Fatigue (AF)** | 0,68 | 0,67 | 1 | 0,98 | 0,9 | 0,97 |
| **Nausea and Vomiting (NV)** | 0,16 | 0,18 | 0,37 | 0,52 | 0,39 | 0,61 |
| **Pain (BP)** | 0,25 | 0,28 | 0,33 | 0,4 | 0,41 | 0,52 |
| **Dyspnea (DY)** | 0,17 | 0,17 | 0,31 | 0,3 | 0,45 | 0,47 |
| **Sleep disturbance (SL)** | 0,45 | 0,46 | 0,59 | 0,66 | 0,69 | 0,99 |
| **Appetite (AP)** | 0,35 | 0,36 | 0,56 | 0,72 | 0,45 | 0,5 |

To calculate the minimally important difference, the standard error of the mean (SEM) has been calculated and the reliability of the scale has been estimated using Cronbach's alpha coefficient. Taking as reference the SEM and the difference between the baseline and final scores of each scale, three groups were classified: improvement (d>SEM), no change (d =+-SEM) worsening (d<-SEM) (See Table 14).

Table 14. Standard Error of the Mean (SEM)

|  | PF | RF | EF | CF | SF | QL | FA | NV |
|---|---|---|---|---|---|---|---|---|
| **EFFECT SIZE** | 0,7 | 0,6 | 0,6 | 0,2 | 0,3 | 0,6 | 1 | 0,4 |
| **STAND. MEAN RESP.** | 0,7 | 0,7 | 0,7 | 0,2 | 0,3 | 0,7 | 1 | 0,5 |
| **SEM** | 15 | 13 | 13 | 15 | 17 | 8 | 11 | 15 |
| **IMPROVED** | 19 | 18 | 19 | 12 | 8 | 21 | 26 | 13 |
| % that improved | 56% | 53% | 56% | 35% | 24% | 62% | 76% | 38% |
| **WORSENED** | 2 | 1 | 3 | 5 | 1 | 6 | 3 | 3 |
| % that worsened | 5,90 % | 2,90 % | 8,80 % | 14,70 % | 2,90 % | 17,60 % | 8,80% | 8,80 % |
| **UNCHANGED** | 13 | 15 | 12 | 7 | 25 | 7 | 5 | 18 |
|  |  |  |  |  |  |  |  |  |
|  | NV | PA | DY | SL | AP | CO | DI | FI |
| **EFFECT SIZE** | 0,4 | 0,3 | 0,3 | 0,6 | 0,6 | 0,3 | 0,1 | 0,1 |
| **STAND. MEAN RESP.** | 0,5 | 0,4 | 0,3 | 0,7 | 0,7 | 0,4 | 0,1 | 0,1 |
| **SEM** | 15 | 16 | 19 | 19 | 19 | 19 | 19 | 17 |
| **IMPROVED** | 13 | 17 | 9 | 19 | 18 | 10 | 6 | 3 |
| % that improved | 38% | 50% | 26% | 56% | 53% | 29% | 18% | 9% |
| **WORSENED** | 3 | 3 | 3 | 4 | 3 | 4 | 5 | 2 |
| % that worsened | 8,80 % | 8,80 % | 8,80 % | 11,80 % | 8,80 % | 11,80 % | 14,70 % | 5,90 % |

(continued)

|  | NV | PA | DY | SL | AP | CO | DI | FI |
|---|---|---|---|---|---|---|---|---|
| **UNCHANGED** | 18 | 14 | 22 | 11 | 13 | 20 | 23 | 29 |

• **Conclusions:**

Treatment with the postbiotic composition of the invention is associated with an improvement in the quality of life (EORTC questionnaire QLQ-C30) of patients with advanced cancer in a statistically significant way ($p<0.01$ to $p<0.005$). The magnitude of the effect is important or very important for most of the scales analyzed. The symptoms that show the greatest improvement are asthenia, appetite, pain, and sleep disturbance. Dyspnea and diarrhea, despite not presenting a significant association, are the symptoms that have presented the greatest magnitude of effect to treatment. The treatment with the composition of the invention has enabled to better tolerate chemotherapeutic treatments, even allowing the re-administration of chemotherapy to patients who had abandoned the therapy due to its toxicity.

Ultimately, the present invention demonstrates that the disclosed postbiotic composition administered to terminal cancer patients is accompanied by an improvement in quality of life, tolerance to chemotherapy, asthenia, and cachexia. These beneficial effects mean that patients' life expectancy appears to increase.

## Claims

1. A postbiotic composition of oral administration for use in the treatment of solid tumors in a patient, caracterized by the fact that said composition comprises lysates of probiotic microorganisms in an amount in percentage by weight with respect to the total weight of the lysates of microorganisms of the composition:

   - 6% to 19% of bacterial lysates of the genus *Bacillus*;
   - 4% to 8% of bacterial lysates of the genus *Bifidobacterium*;
   - 15% to 25% of bacterial lysates of the genus *Lactobacillus*;
   - 50% to 60% of yeast lysates of the genus *Saccharomyces*;
   - 1.5% to 5% of bacterial lysates of the genus *Streptococcus*.

2. The composition, according to the preceding claim, comprising lysates of probiotic microorganisms in an amount in percentage by weight with respect to the total weight of the lysates of microorganisms of the composition:

   - 6% to 19% of bacterial lysates of the genus *Bacillus*;
   - 4% to 8% of bacterial lysates of *Bifidobacterium lactis*,
   - 15% to 25% of bacterial lysates of the genus *Lactobacillus*;
   - 50% to 60% of yeast lysates of the genus *Saccharomyces*;
   - 1.5% to 5% of bacterial lysates of the genus *Streptococcus*.

3. The composition, according to any of the preceding claims, comprising lysates of probiotic microorganisms in an amount as a percentage by weight with respect to the total weight of the lysates of microorganisms of the composition:

   - 6% to 19% of bacterial lysates of *Bacillus clausii, Bacillus coagulans, Bacillus licheniformis, Bacillus subtilis* and *Bacillus mesentericus*;
   - 4% to 8% of bacterial lysates of *Bifidobacterium lactis*;
   - 15% to 25% of bacterial lysates of *Lactobacillus acidophilus, Lactobacillus bulgaricus, Lactobacillus casei, Lactobacillus fermentum, Lactobacillus reuteri* and *Lactobacillus rhamnosus*;
   - 50% to 60% of yeast lysates of *Saccharomyces cerevisiae*;
   - 1.5% to 5% of bacterial lysates of *Streptococcus thermophilus*.

4. The composition, according to any of the preceding claims, comprising lysates of probiotic microorganisms in an amount as a percentage by weight with respect to the total weight of the lysates of microorganisms of the composition:

   - 6% to 19% of bacterial lysates of *Bacillus licheniformis, Bacillus pumilus* and *Bacillus subtilis,* the percentage by weight of *Bacillus licheniformis, Bacillus pumilus* and *Bacillus subtilis* being approximately the same;
   - 4% to 8% of bacterial lysates of *Bifidobacterium lactis*;

- 15% to 25% of bacterial lysates of *Lactobacillus acidophilus, Lactobacillus casei, Lactobacillus fermentum, Lactobacillus reuteri, Lactobacillus rhamnosus* and *Lactobacillus bulgaricus,* the percentage by weight of *Lactobacillus casei* being higher than that of *Lactobacillus acidophilus,* which in turn is superior to that of *Lactobacillus bulgaricus,* which in turn is superior to that of *Lactobacillus fermentum, Lactobacillus reuteri* and *Lactobacillus rhamnosus,* the percentage by weight of these 3 strains being approximately identical;
- 50% to 60% of yeast lysates of *Saccharomyces cerevisiae;*
- 1.5% to 5% of bacterial lysates of *Streptococcus thermophilus.*

5. The composition, according to any of the preceding claims, which does not include bacterial lysates of the genera *Akkermansia, Enterococcus* and *Escherichia.*

6. The composition, according to any of the preceding claims, comprising one or more of the following additives: resveratrol, astaxanthin, bromelain, papain, fermented rice starch, corn starch and FOS.

7. The composition, according to any of the preceding claims, which is presented in the form of dry powder.

8. The composition, according to any of the preceding claims, wherein the treatment of solid tumors includes the improvement of quality of life in the patient.

9. The composition, according to the preceding claim, wherein the improvement in the quality of life concerns one or more of the following symptoms: anxiety, tiredness, nausea, vomiting, pain, difficulty sleeping, stress, asthenia, appetite, pain, dyspnea and diarrhea.

10. The composition, according to any of the preceding claims, wherein the solid tumor is one or more of the following: prostate cancer, breast cancer, cancer of the central nervous system (CNS), pancreatic cancer, esophageal cancer, uterine cancer, lymphoma, pheochromocytoma and sarcoma.

11. The composition, according to any of the preceding claims, wherein the patient is a terminal cancer patient.

12. The composition, according to any of the preceding claims, wherein the composition is administered in an amount between 100mg to 700mg per dose, and between 1 to 6 doses per day for at least 2 weeks.

13. The composition, according to any of the preceding claims, wherein the treatment is performed before, simultaneously or after the treatment of the patient with chemotherapy or radiotherapy.

14. The composition, according to any of the preceding claims, wherein the amount of the composition is different each time it is administered to the patient during the day.

15. A pharmaceutical composition comprising an effective pharmaceutical amount of the composition, in accordance with any of claims 1 to 14, and a pharmaceutically acceptable excipient.

Figure 1

Figure 2. a

Figure 2. b

Figure 2. c

Figure 2. d

Figure 3

Figure 4

Figure 5

Figure 6

Europäisches
Patentamt

European
Patent Office

Office européen
des brevets

## EUROPEAN SEARCH REPORT

Application Number

EP 23 38 2314

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ES 2 783 885 A1 (IGEN BIOLAB GROUP AG [CH]) 18 September 2020 (2020-09-18) | 15 | INV. A61K35/74 |
| Y | * the whole document * | 1-15 | A23L33/135 A61K9/00 |
| A,D | WO 2019/169179 A1 (SHAFER KIM [US]) 6 September 2019 (2019-09-06) * see claims and examples * | 1-15 | A61K35/742 A61K35/745 A61K35/747 A61P35/00 |
| Y | WO 2022/068924 A1 (CHENGDU KUACHANGAOPU MEDICAL TECHNOLOGY) 7 April 2022 (2022-04-07) * see claims, Tables 2 and 3, examples 3-5 * | 1-15 | |
| Y | Anonymous: "Record History ¦ ver. 8: 2022-06-28 ¦ NCT04009122 ¦ ClinicalTrials.gov. Evaluation of the Quality of Life of Patients With Metastatic Non-small Cell Lung Cancer With Supplementary Therapy With IGEN-0206", , 28 June 2022 (2022-06-28), XP093132879, Retrieved from the Internet: URL:https://clinicaltrials.gov/study/NCT04009122?tab=history&a=8 [retrieved on 2024-02-19] * the whole document * | 1-15 | TECHNICAL FIELDS SEARCHED (IPC) A61K A23L A61P |

-/--

The present search report has been drawn up for all claims

| Place of search | Date of completion of the search | Examiner |
|---|---|---|
| Munich | 23 February 2024 | Merckling-Ruiz, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

page 1 of 4

## EUROPEAN SEARCH REPORT

Application Number

EP 23 38 2314

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SONG DINGKA ET AL: "Beneficial insights into postbiotics against colorectal cancer", FRONTIERS IN NUTRITION, vol. 10, 10 March 2023 (2023-03-10), XP093111686, Lausanne ISSN: 2296-861X, DOI: 10.3389/fnut.2023.1111872 Retrieved from the Internet: URL:https://www.ncbi.nlm.nih.gov/pmc/artic les/PMC10036377/pdf/fnut-10-1111872.pdf> * the whole document * | 1-15 | |
| E | FR 3 133 727 A3 (IGEN BIOLAB GROUP AG [CH]) 29 September 2023 (2023-09-29) * see claims * | 15 | |
| Y | WO 2023/021141 A1 (NESTLE SA [CH]) 23 February 2023 (2023-02-23) * see claims * | 1-15 | |
| Y | WO 2017/083470 A1 (MCKENNA ELIZABETH [US]) 18 May 2017 (2017-05-18) * see claims * | 1-15 | TECHNICAL FIELDS SEARCHED (IPC) |

-/--

The present search report has been drawn up for all claims

| Place of search | Date of completion of the search | Examiner |
|---|---|---|
| Munich | 23 February 2024 | Merckling-Ruiz, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EUROPEAN SEARCH REPORT

Application Number

EP 23 38 2314

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZHUO QIAN ET AL: "Lysates of Lactobacillus acidophilus combined with CTLA-4-blocking antibodies enhance antitumor immunity in a mouse colon cancer model", SCIENTIFIC REPORTS, vol. 9, no. 1, 27 December 2019 (2019-12-27), XP093111685, US ISSN: 2045-2322, DOI: 10.1038/s41598-019-56661-y Retrieved from the Internet: URL:https://www.nature.com/articles/s41598-019-56661-y> * abstract * | 1-15 | |
| Y | Sukjin Kim(???) ET AL: "Postbiotics for cancer prevention and treatment", ??????, 1 January 2021 (2021-01-01), pages 142-153, XP093111684, DOI: 10.7845/kjm.2021.1067 Retrieved from the Internet: URL:http://www.kjom.org/journal/view.html?doi=10.7845/kjm.2021.1067 [retrieved on 2023-12-13] * the whole document * | 1-15 | |

-/--

TECHNICAL FIELDS SEARCHED (IPC)

The present search report has been drawn up for all claims

| Place of search | Date of completion of the search | Examiner |
|---|---|---|
| Munich | 23 February 2024 | Merckling-Ruiz, V |

EPO FORM 1503 03.82 (P04C01)

## EUROPEAN SEARCH REPORT

Application Number

EP 23 38 2314

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HOMAYOUNI RAD AZIZ ET AL: "Postbiotics as Promising Tools for Cancer Adjuvant Therapy", ADVANCED PHARMACEUTICAL BULLETIN, vol. 11, no. 1, 1 January 2021 (2021-01-01), pages 1-5, XP093111680, Iran ISSN: 2228-5881, DOI: 10.34172/apb.2021.007 Retrieved from the Internet: URL:https://www.ncbi.nlm.nih.gov/pmc/articles/PMC7961229/pdf/apb-11-1.pdf> * see abstract and Table 1 * ----- | 1-15 | |
| Y | ES 2 264 368 A1 (EXPOSITO MESA FRANCISCO [ES]) 16 December 2006 (2006-12-16) * see claims * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 23 February 2024 | Merckling-Ruiz, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 4

## ANNEX TO THE EUROPEAN SEARCH REPORT
## ON EUROPEAN PATENT APPLICATION NO.

EP 23 38 2314

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2024

| Patent document cited in search report | | Publication date | Patent family member(s) | | Publication date |
|---|---|---|---|---|---|
| ES 2783885 | A1 | 18-09-2020 | ES | 2783723 A1 | 17-09-2020 |
| | | | ES | 2783885 A1 | 18-09-2020 |
| WO 2019169179 | A1 | 06-09-2019 | NONE | | |
| WO 2022068924 | A1 | 07-04-2022 | EP | 4223300 A1 | 09-08-2023 |
| | | | JP | 2023544310 A | 23-10-2023 |
| | | | US | 2023405063 A1 | 21-12-2023 |
| | | | WO | 2022068924 A1 | 07-04-2022 |
| FR 3133727 | A3 | 29-09-2023 | ES | 2952438 A1 | 31-10-2023 |
| | | | FR | 3133727 A3 | 29-09-2023 |
| | | | WO | 2023180805 A1 | 28-09-2023 |
| WO 2023021141 | A1 | 23-02-2023 | AU | 2022331116 A1 | 18-01-2024 |
| | | | CA | 3225609 A1 | 23-02-2023 |
| | | | WO | 2023021141 A1 | 23-02-2023 |
| WO 2017083470 | A1 | 18-05-2017 | AR | 106660 A1 | 07-02-2018 |
| | | | AU | 2016352986 A1 | 28-06-2018 |
| | | | AU | 2023251534 A1 | 16-11-2023 |
| | | | BR | 112018009437 A2 | 04-12-2018 |
| | | | CA | 3004951 A1 | 18-05-2017 |
| | | | CN | 108430482 A | 21-08-2018 |
| | | | EA | 201891142 A1 | 30-11-2018 |
| | | | EP | 3373943 A1 | 19-09-2018 |
| | | | HK | 1252018 A1 | 10-05-2019 |
| | | | IL | 259228 A | 31-07-2018 |
| | | | JP | 2018532814 A | 08-11-2018 |
| | | | JP | 2023011830 A | 24-01-2023 |
| | | | SG | 10202112466U A | 30-12-2021 |
| | | | SG | 11201803906P A | 28-06-2018 |
| | | | WO | 2017083470 A1 | 18-05-2017 |
| ES 2264368 | A1 | 16-12-2006 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 114847483 A
- CN 102475776 A
- WO 2019169179 A1
- ES 201930242 P
- ES 201930280 P

**Non-patent literature cited in the description**

- **LU K ; DONG S ; WU X ; JIN R ; CHEN H.** *Probiotics in Cancer. Front. Oncol.,* 2021, vol. 11, 638148